(19) Europäisches Patentamt
European Patent Office
Office européen des brevets

(11) Veröffentlichungsnummer: **0 510 445 B1**

# (12) EUROPÄISCHE PATENTSCHRIFT

(45) Veröffentlichungstag der Patentschrift: **09.08.95**

(51) Int. Cl.[6]: **C07C 309/73**, C07C 309/66, C07D 207/40, G03F 7/004

(21) Anmeldenummer: **92106141.2**

(22) Anmeldetag: **09.04.92**

## (54) Säurespaltbare strahlungsempfindliche Verbindungen, diese enthaltendes strahlungsempfindliches Gemisch und daraus hergestelltes strahlungsempfindliches Aufzeichnungsmaterial.

(30) Priorität: **20.04.91 DE 4112970**

(43) Veröffentlichungstag der Anmeldung:
**28.10.92 Patentblatt 92/44**

(45) Bekanntmachung des Hinweises auf die Patenterteilung:
**09.08.95 Patentblatt 95/32**

(84) Benannte Vertragsstaaten:
**DE FR GB IT**

(56) Entgegenhaltungen:
**EP-A- 0 297 443**
**EP-A- 0 315 748**

**CHEMICAL ABSTRACTS, Band 113, Nr. 17, 22. Okfober 1990, Seite 842, Spalte 1, Zusammenfassung Nr. 153046W, Columbus, Ohio, US; & EP-A-353 732 (CIBA-GEIGY AG) 07-02-1990**

(73) Patentinhaber: **HOECHST AKTIENGESELLSCHAFT**

**D-65926 Frankfurt am Main (DE)**

(72) Erfinder: **Pawlowski, Georg, Dr.**
**Fritz-Kalle-Strasse 34**
**W-6200 Wiesbaden (DE)**
Erfinder: **Dammel, Ralph, Dr.**
**70 Wood Cove Drive,**
**Wood Estates**
**Coventry,**
**Rhode Island 02816 (US)**
Erfinder: **Röschert, Horst, Dr.**
**Am Pfingstborn 16**
**W-6531 Ober Hilbersheim (DE)**
Erfinder: **Spiess, Walter, Dr.**
**Rheingaustrasse 20**
**W-6110 Dieburg (DE)**
Erfinder: **Eckes, Dl. Charlotte**
**Zugspitzstrasse 15**
**6200 Wiesbaden-Dotzheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

JOURNAL OF ORGANIC CHEMISTRY OF THE USSR, Band 23, Nr. 11, November 1987, Seiten 2017-2021; V.N. ODINOKOV et al.: "Ozonolysis of alkenes and reactions of polyfunctional compounds. XXXII. Synthesis of (xi)-3,7-dimethyl-2,7-octadienyl propionate"

CHEMICAL ABSTRACTS, Band 107, Nr. 11, 14. September 1987, Seite 674, Spalte 1, Zusammenfassung Nr. 96539j, Columbus, Ohio, US; J.D. STEWART et al.: "Preparation of alpha-arylsulfonyl lactams", & HETEROCYCLES 1987, 25(1), 213-16

CHEMICAL ABSTRACTS, Band 102, Nr. 11, 18. März 1985, Seite 553, Spalte 2, Zusammenfassung Nr. 95437j, Columbus, Ohio, US; G.G. BALEZINA et al.: "Insect pheromones and their analogs. IX. Stereospecific synthesis of (Z)-9,11-dodecadienyl acetate - a component of the sex pheromone of Diparopsis cactanea", & KHIM. PRIR. SOEDIN. 1984, (3), 378-82

THE JOURNAL OF ORGANIC CHEMISTRY, Band 49, Nr. 23, 16. November 1984, Seiten 4545-4547, Easton, US; D.A. JAEGER et al.: ""Destructible" surfactans based on a ketal group"

JOURNAL OF THE CHEMICAL SOCIETY, PERKIN TRANSACTIONS I, Nr. 12, 1983, Seiten 2963-2966, Letchworth, GB; J.N. NAVALKISHORE et al.: "Convenient synthetic route to 6,8-dioxabicyclo[3.2.1]octanes, the aggregation pheromone components of Bark Beetles"

THE JOURNAL OF ORGANIC CHEMISTRY, Band 48, Nr. 17, 26. August 1983, Seiten 2887-2891, Easton, US; X. CREARY et al.: "Reactions of triflates with potassium diethyl phosphite. Formation of phosphate esters"

THE JOURNAL OF ORGANIC CHEMISTRY, Band 48, Nr. 1, 14. Januar 1983, Seiten 31-33, Easton, US; T. KAMETANI et al.: "New construction of a steroidal ring system. Stereoselective synthesis of (+)-androstane-2,17-dione"

TETRAHEDRON LETTERS, Band 21, Nr. 15, 1980, Seiten 1479-1482, Oxford, GB; K. TATSUTA et al.: "Stereospecific total synthesis and absolute configuration of a macrocyclic lactone antibiotic, A26771B"

JOURNAL OF ORGANIC CHEMISTRY, Band 45, Nr. 3, 1. Februar 1980, Seiten 501-506, Easton, US; W.K. ANDERSON et al.: "Synthesis of C-ring-functionalized A-ring-aromatic trichothecane analogues"

CHEMICAL ABSTRACTS, Band 91, Nr. 13, 24. September 1979, Seite 587, Spalte 1, Zusammenfassung Nr. 108010w, Columbus, Ohio, US; & JP-A-79 44 698 (ASAHI CHEMICAL INDUSTRY CO., LTD) 09-04-1979

CHEMICAL ABSTRACTS, Band 89, Nr. 14, 2. Oktober 1978, Seite 41, Spalte 2, Zusammenfassung Nr. 110919b, Columbus, Ohio, US; Y.N. BELOKON et al.: "Synthesis of crosslinked polyacrylamide gel containing salicylaldehyde fragments",& VYSOKOMOL. SOEDIN., SER. A 1978, 20(6), 1304-8

BULLETIN OF THE CHEMICAL SOCIETY OF JAPAN, Band 51, Nr. 2, Februar 1978, Seiten 547-549; J. TSUJI et al.: "A modified synthesis of 2,15-hexadecanedione, a precursor of muscone, from a butadiene telomer"

THE JOURNAL OF ORGANIC CHEMISTRY, Band 42, Nr. 1, 7. Januar 1977, Seiten 118-125, Easton, US; W.J. GENSLER et al.: "Synthesis of DL-methyl meromycolate"

THE JOURNAL OF ORGANIC CHEMISTRY, Band 41, Nr. 1, 9. Januar 1976, Seiten 136-141, Easton, US; W.C. AGOSTA et al.: "Loss of water from ketones in isobutane chemical ionization mass spectrometry"

CHEMICAL ABSTRACTS, Band 84, Nr. 3, 19. Januar 1976, Seite 493, Spalte 1, Zusammenfassung Nr. 17573w, Columbus, Ohio, US; O.P. VIG et al.: "Terpenoids. CIV. New synthesis of caparrapidiol", & INDIAN J. CHEM. 1975, 13(10), 1003-4

CHEMICAL ABSTRACTS, Band 83, Nr. 23, 8. Dezember 1975, Seite 389, Spalte 1, Zusammenfassung Nr. 192488y, Columbus, Ohio, US; O.P. VIG et al.: "New synthesis of 9-keto-trans-2-deconoic acid", & J. INDIAN CHEM. SOC. 1975, 52(6), 543-5

CHEMICAL ABSTRACTS, Band 83, Nr. 21, 24. November 1975, Seite 568, Spalte 1, Zusammenfassung Nr. 178949t, Columbus, Ohio, US; I. FLEMING et al.: "Protected 5-hydroxypentanal synthon", & SYNTH. COMMUN. 1975, 5(3), 177-80

THE JOURNAL OF ORGANIC CHEMISTRY,

**Band 35, Nr. 4, April 1970, Seiten 973-978, Easton, US; J.R. HAZEN: "Methoxonium ions in solvolysis. Neighboring acetal participation"**

**TETRAHEDRON, (INCL. TETRAHEDRON REPORTS), Band 25, Nr. 15, August 1969, Seiten 3017-3031, Oxford GB; N. TSUJI et al.: "Studies on julimycins-V. The configurations and conformations of julichrome O1-3 and its C9-epimer"**

**JOURNAL OF MEDICINAL CHEMISTRY, Band 10, Nr. 6, November 1967, Seiten 1088-1091, Washington, DC, US; J.P. YARDLEY et al.: "Synthesis and amebicidal activities of some 1',2'-secoemetine derivatives"**

**MICROELECTRONIC ENGINEERING, Band 13, Nr. 1/4, März 1991, Seiten 33-36, Amsterdam, NL; L. SCHLEGEL et al.: "Highly sensitive positive deep UV resist utilizing a sulfonate acid generator and a tetrahydropyranyl inhibitor"**

**MICROELECTRONIC ENGINEERING, Band 13, Nr. 1/4, März 1991, Seiten 3-10, Amsterdam, NL; E. REICHMANIS et al.: "Chemistry and process for deep-UV resists"**

## Beschreibung

Die Erfindung betrifft strahlungsempfindliche und säurespaltbare Verbindungen sowie ein positiv arbeitendes, d.h. durch Bestrahlung löslich werdendes strahlungsempfindliches Gemisch, das diese Verbindungen enthält. Das Gemisch enthält

(a) ein in Wasser unlösliches, in wäßrig-alkalischen Lösungen lösliches oder zumindest quellbares Bindemittel und

(b) eine Verbindung, die unter Einwirkung von aktinischer Strahlung eine starke Säure bildet und die mindestens eine durch Säure spaltbare C-O-C-Bindung aufweist.

Die Erfindung betrifft weiterhin ein daraus hergestelltes strahlungsempfindliches Aufzeichnungsmaterial, das zur Herstellung von Photoresists, elektronischen Bauteilen, Druckplatten oder zum Formteilätzen geeignet ist.

Die stetige Verkleinerung der Strukturen, beispielsweise in der Chip-Herstellung bis in den Bereich von weniger als 1 µm, erfordert veränderte lithographische Techniken. Um solche feinen Strukturen abzubilden, verwendet man Strahlung mit einer kurzen Wellenlänge, wie energiereiches UV-Licht, Elektronen- und Röntgenstrahlen. Das strahlungsempfindliche Gemisch muß an die kurzwellige Strahlung angepaßt sein. Eine Zusammenstellung der an das strahlungsempfindliche Gemisch gestellten Anforderungen ist in dem Aufsatz von C.G. Willson "Organic Resist Materials - Theory and Chemistry" [Introduction to Microlithography, Theory, Materials, and Processing, Herausgeber L.F. Thompson, C.G. Willson, M.J. Bowden, ACS Symp. Ser., 219, 87 (1983), American Chemical Society, Washington] angegeben. Es bestand daher ein verstärkter Bedarf an strahlungsempfindlichen Gemischen, die in den neueren Technologien, wie der Mid- oder Deep-UV-Lithographie, [Belichtung z. B. mit Excimer-Lasern bei Wellenlängen von 305 nm (XeF), 248 nm (KrF), 193 nm (ArF)], der Elektronen- oder der Röntgenstrahl-Lithographie, einsetzbar sind, vorzugsweise darüber hinaus in einem weiten Spektralbereich empfindlich sind und dementsprechend auch in der konventionellen UV-Lithographie verwendet werden können.

Positiv arbeitende strahlungsempfindliche Gemische zur Herstellung strahlungsempfindlicher Aufzeichnungsmaterialien sind bekannt. Handelsüblich sind Gemische, die o-Chinon-diazid-Derivate in wäßrig-alkalisch löslichen Bindemitteln, beispielsweise Novolaken oder Polyhydroxystyrolen, enthalten. Die Empfindlichkeit dieser Materialien gegenüber aktinischer, insbesondere aber hochenergetischer, kurzwelliger Strahlung, wie Licht eines KrF-Excimer-Lasers mit einer Wellenlänge von 248 nm oder Elektronenstrahlung, ist aber unzureichend.

Weiterhin sind positiv arbeitende strahlungsempfindliche Gemische bekannt, bei denen durch Einwirkung von aktinischer Strahlung auf einen in dieser Mischung enthaltenen Photoinitiator eine Säure gebildet wird und diese dann in einer Folgereaktion eine ebenfalls in der Mischung enthaltene säurespaltbare Verbindung in den bestrahlten Bereichen gegenüber einem entsprechenden, bevorzugt wäßrigalkalischen Entwickler löslich macht. Derartige Materialien zeichnen sich im allgemeinen durch eine verbesserte Empfindlichkeit gegenüber aktinischer Strahlung aus.

Es sind zahlreiche Gemische bekannt, die als wesentliche Komponenten ein polymeres, in wäßrig-alkalischen Lösungen lösliches Bindemittel, eine löslichkeitsinhibierende Verbindung und eine Verbindung, die bei Bestrahlung die zur Spaltung erforderliche Säure liefert, bekannt. Das Bindemittel ist zumeist ein Novolak-Harz. Viele dieser Gemische besitzen eine hohe Empfindlichkeit gegenüber aktinischer Strahlung. Sie werden als chemisch verstärkte, photokatalytische 3-Komponentensysteme bezeichnet.

Von diesen Gemischen haben insbesondere solche eine gewisse kommerzielle Bedeutung erlangt, deren säurespaltbare Komponente eine oder mehrere Acetaleinheiten aufweist. Diese Gemische haben jedoch gewisse Nachteile. So besitzen sie auf den Substratmaterialien, auf die sie aufgebracht werden müssen, eine nur begrenzte Stabilität, was zu einer unzureichenden, nicht reproduzierbaren Wiedergage der Bildvorlage führt. Dies kann nur durch Einführung zusätzlicher Schutzschichten, z. B. gemäß DE-A 36 21 376 (= US-A 4 840 867), verbessert werden. Die Ursachen der Verschlechterung der Bildwiedergabe sind nicht im einzelnen bekannt und nur unzureichend untersucht. Beispielsweise ist das Prozeßfenster, d. h. der Verarbeitungsspielraum, für die Belichtung dieser Gemische sehr schmal und häufig nicht eindeutig reproduzierbar. Insbesondere hängt die Qualität der Bildwiedergabe stark von der Zeitdifferenz zwischen Belichtung und Entwicklung, der sogenannten "delay time" ab. Grundsätzlich ist davon auszugehen, daß Diffusionsvorgänge, die zu diesem Verhalten führen, nicht einfach steuerbar sind. Es läßt sich jedoch zusätzlich vermuten, daß es während der Trocknung des Gemischs auf einem Substratmaterial zu einer partiellen Verdampfung des Photoinitiators oder der säurelabilen Verbindung oder zu einer Entmischung der einzelnen Gemischbestandteile kommt, was besonders häufig bei säurelabilen Verbindungen mit geringer Löslichkeit in den üblichen Beschichtungslösemitteln beobachtet wird.

Ferner ist durch die Arbeiten von C.C. Petropoulos [J. Polym. Sci., Polym. Chem. Ed., 15, 1637 (1977)] bekannt, daß aromatische Acetale, die in Nachbarstellung eine Nitrogruppe tragen, ohne Säurekatalyse durch energiereiche UV-Strahlung photozersetzbar sind, und in positiv arbeitenden strahlungsempfindlichen Aufzeichnungsmaterialien Verwendung finden können. Die Photoempfindlichkeit dieser Verbindungen ist jedoch für praktische Anwendungen unzureichend, da ihre Lichtreaktion nicht chemisch verstärkt werden kann.

In der DE-A 37 21 741 (= US-A 4 883 740) werden strahlungsempfindliche Gemische beschrieben, die ein wasserunlösliches, in wäßrig-alkalischen Lösungen lösliches polymeres Bindemittel und eine organische Verbindung enthalten, die mindestens eine durch Säure spaltbare Gruppierung sowie eine Gruppierung enthält, die unter Einwirkung von Strahlung eine starke Säure bildet. Als strahlungsempfindliche Gruppen werden ausschließlich Oniumsalzgruppen, insbesondere Sulfoniumsalzgruppen, beschrieben.

Die Verwendung von Onium-Salzen, wie Diazonium-, Phosphonium-, Sulfonium- und Jodonium-Salze von nicht nucleophilen Säuren, wie $HSbF_6$, $HAsF_6$ oder $HPF_6$ als photolytische Säurebildner, bringt Nachteile mit sich, die ihre Einsatzmöglichkeiten in verschiedenen Anwendungsbereichen drastisch einschränken. So sind z.B. viele der Oniumsalze toxisch. Ihre Löslichkeit ist in vielen Lösemitteln unzureichend, weshalb nur wenige Lösemittel zur Herstellung einer Beschichtungslösung geeignet sind. Darüber hinaus werden bei Verwendung der Oniumsalze z.T. unerwünschte Fremdatome eingeführt, die insbesondere in der Mikrolithographie zu Prozeßstörungen führen können. Ferner bilden die Oniumsalze bei der Photolyse sehr stark korrodierend wirkende Brönstedt-Säuren. Diese Säuren greifen empfindliche Substrate an, so daß der Einsatz solcher Gemische zu unbefriedigenden Ergebnissen führt. Auch Halogenverbindungen, wie Trichlormethyltriazin-Derivate oder Trichlormethyloxadiazol-Derivate, bilden stark korrosiv wirkende Halogenwasserstoffsäuren.

In neueren Arbeiten von F.M. Houlihan et al., SPIE 920, 67 (1988) wurden anhand positiv arbeitender Systeme gezeigt, daß neben den oben genannten Säurebildnern auch Nitrobenzyltosylate, die bei Belichtung Sulfonsäuren mit geringer Wanderungstendenz bilden, in bestimmten säurelabilen Resistformulierungen verwendbar sind. Es kann aus diesen Ergebnissen abgeleitet werden, daß solche Verbindungen auch für photohärtbare Systeme verwendet werden können. Die dabei erzielten Empfindlichkeiten, insbesondere gegenüber UV-Strahlung von 350 bis 450 nm und die thermische Stabilität der Photoresists erwiesen sich jedoch als unzureichend.

Wegen der aufgeführten Unzulänglichkeiten und Nachteile besteht daher ein Bedarf an weiteren strahlungsempfindlichen Gemischen, die die oben beschriebenen Nachteile nicht aufweisen und eine praxisgerechte Reaktivität besitzen.

Aufgabe der Erfindung war es daher, photolytisch säurebildende und säurespaltbare Verbindungen sowie ein strahlungsempfindliches Gemisch auf deren Basis vorzuschlagen, wobei die photolytisch eine Säure bildende Verbindung möglichst stabil auf allen bekannten Substraten sein sollte und als Photolyseprodukt eine nicht korrosiv wirkende Säure liefert. Darüber hinaus soll mit der Erfindung ein strahlungsempfindliches Gemisch bereitgestellt werden, das insbesondere eine Entmischung der photoaktiven Verbindung und der löslichkeitsdifferenzierenden Verbindung vermeidet.

Gelöst wird die Aufgabe durch strahlungsempfindliche und säurespaltbare Verbindungen der allgemeinen Formel I

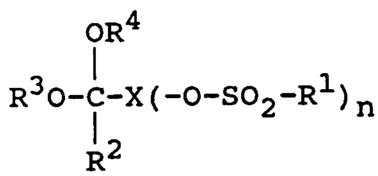

(I)

worin

| | |
|---|---|
| $R^1$ | einen Alkyl-, hochfluorierten Alkyl-, Perfluoralkyl- oder Arylrest, |
| $R^2$ | ein Wasserstoffatom, einen Alkyl- oder Arylrest, eine der Gruppen $(R^1\text{-}SO_2\text{-}O\text{-})_nX$- oder $R^3O$-, |
| $R^3$ und $R^4$ | gleich oder verschieden sind und Alkyl-, Cycloalkylalkyl-, Cycloalkenylalkyl- oder Aralkylreste, in denen 1 bis 3 aliphatische $CH_2$- oder CH-Gruppen durch $NR^5$, O, S, CO, CO-O, CO-NH, O-CO-NH, CO-NH-CO, NH-CO-NH, $SO_2$, $SO_2$-O, $SO_2$-NH ersetzt sein können; Alkenyl-, Alkinyl-, Cycloalkyl- oder Cycloalkenylreste bedeuten oder untereinander zu einem heterocyclischen Ring verbunden sind, |
| $R^5$ | einen Acylrest, |

X eine Alkantriyl-, Cycloalkantriyl- oder Arentriylgruppe oder eine Alkantetrayl-, Cycloalkantetrayl- oder Arentetraylgruppe und

n 2 oder 3

bedeuten.

Bevorzugt sind solche Verbindungen der allgemeinen Formel I, in denen

$R^1$ einen gegebenenfalls hochfluorierten Alkylrest mit 1 bis 6 Kohlenstoffatomen, einen Arylrest mit 6 bis 12 Kohlenstoffatomen und

$R^2$ ein Wasserstoffatom oder einen Rest $R^3O$ bedeutet.

$R^3$ und $R^4$ sind vorzugsweise gleich und bedeuten insbesondere Alkyl-, Cycloalkylalkyl-, Cycloalkenylalkyl- oder Aralkylgruppen mit weniger als 16 Kohlenstoffatomen, wobei gegebenenfalls 1 bis 3 aliphatische $CH_2$- bzw. CH-Gruppen durch $NR^5$, O, S, CO, COO, CONH, OCONH, CONHCO, NHCONH, $SO_2$, $SO_2O$ oder $SO_2NH$ ersetzt sind; Alkenyl-, Alkinyl-, Cycloalkyl- oder Cycloalkenylreste.

Wenn $R^3$ und $R^4$ gemeinsam einen heterocyclischen Ring bilden, ist es bevorzugt ein 1,3-Dioxolan- oder 1,3-Dioxanring.

$R^5$ kann insbesondere ein Alkanoyl-, Aroyl-, ein Alkyl-, Alkenyl-, Aralkyl- oder Arylsulfonyl-, ein Alkyl-, Alkenyl-, Aralkyl- oder Arylphosphonylrest mit bis zu 12 Kohlenstoffatomen, besonders bevorzugt ein Alkanoylrest mit 2 bis 6 oder ein Aroylrest mit 7 bis 10 Kohlenstoffatomen sein.

X ist bevorzugt eine mit 1 bis 3 Sulfonyloxygruppen $R^1$-$SO_2$-O- substituierte Alkylgruppe mit 2 bis 10 Kohlenstoffatomen, eine Cycloalkylgruppe mit 4 bis 10 Kohlenstoffatomen oder eine Arylgruppe mit 6 bis 12 Kohlenstoffatomen.

Als Aryl- bzw. Arylengruppen kommen sowohl unsubstituierte als auch durch Halogenatome, Alkyl-, Alkoxy-, Nitro- oder Cyanogruppen substituierte Gruppen in Betracht. Unter Aralkylgruppen sollen solche verstanden werden, die 1 bis 2 aromatische Reste enthalten, die durch aliphatische Gruppierungen verbunden und über ein aliphatisches Kohlenstoffatom gebunden sind.

Besonders bevorzugt sind solche Verbindungen der allgemeinen Formel I, in denen

$R^1$ einen Methyl-, Ethyl-, Trifluormethyl- oder Hexafluorisopropylrest oder einen gegebenenfalls durch 1 bis 3 Alkyl- oder Alkoxygruppen mit 1 bis 3 Kohlenstoffatomen, 1 bis 3 Halogenatome, 1 oder 2 Nitro-, Cyano- oder Trifluormethylgruppen oder entsprechende Kombinationen substituierten Phenylrest darstellt und

X eine mit 1 bis 3 Sulfonyloxygruppen $R^1$-$SO_2$-O- substituierte Arylgruppe ist.

Ganz besonders bevorzugt sind solche Verbindungen der allgemeinen Formel I, in denen

$R^2$ ein Wasserstoffatom und

X einen mit 1 bis 3 Sulfonyloxygruppen $R^1$-$SO_2$-O- substituierten aromatischen Sechsring bedeutet.

Die Herstellungsweise solcher Verbindungen ist an sich bekannt. Sie kann beispielsweise analog zu den in der EP-A 0 312 751 angegebenen Synthesevorschriften erfolgen, wobei in einer Vorstufe das entsprechende Sulfonsäureesterderivat hergestellt werden muß. Weitere geeignete Synthesevorschriften sind je nach Art der Substituenten $R^2$, $R^3$ und $R^4$ der DE-A 26 10 842 zu entnehmen. Im Folgenden wird die Synthese typischer und bevorzugter Vertreter dieser Verbindungsklassen an einzelnen Beispielen beschrieben.

Herstellungsbeispiel 1:

1. Stufe: 80,6 g (0,66 mol) 4-Hydroxy-benzaldehyd wurden in 200 ml Tetrahydrofuran gelöst, mit 138 ml Triethylamin versetzt und auf -5 °C abgekühlt. Dazu wurden 125,8 g (0,66 mol) p-Toluolsulfonsäurechlorid, gelöst in 330 ml Tetrahydrofuran und auf -5 °C vorgekühlt, bei -8 bis - 5 °C zugetropft. Es wurde 1 Stunde bei Raumtemperatur nachgerührt. Die Lösung wurde in 1000 ml destilliertes Wasser eingegossen und die Mischung mit konzentrierter Salzsäure auf pH 2 gebracht. Das ausgefallene Öl wurde in Ether aufgenommen und die wäßrige Phase mit Ether extrahiert. Die vereinigten organischen Phasen wurden mit Wasser gewaschen und getrocknet. Nach dem Einengen verblieb ein Rückstand, der aus Cyclohexan/Methylenchlorid umkristallisiert wurde. Es wurden 120 g (66,1%) 4-(Toluol-4-sulfonyloxy)-benzaldehyd (weißer Feststoff mit einem Schmelzpunkt von 70 bis 72 °C) erhalten.

2. Stufe: 7,2 g (0,026 mol) 4-(Toluol-4-sulfonyloxy)-benzaldehyd wurden zusammen mit 7,2 g (0,052 mol) Phenoxyethanol, 4,25 g (0,0286 mol) Orthoameisensäuretriethylester und 100 mg p-Toluolsulfonsäure in 150 ml Toluol zum Rückfluß erwärmt. Nach etwa 2 Stunden wurde ein Teil des Destillats abgenommen, bis die Kopftemperatur der Siedetemperatur des reinen Toluols entsprach. Dieser Vorgang wurde so lange wiederholt, bis die Kopftemperatur nicht mehr unter den Siedepunkt von reinem Toluol sank. Nach

weiteren 2 Stunden wurde die Destillation für etwa 45 Minuten durch Anlegen eines Vakuums von 4 Torr unterstützt und alle flüchtigen Bestandteile abdestilliert. Der erkaltete Rückstand wurde in Methylenchlorid aufgenommen, je zweimal mit 2%iger Natronlauge und vollentsalztem Wasser gewaschen und die organische Phase getrocknet. Nach Entfernung des Lösemittels verblieben 12,9 g (93%) eines fahlgelben Öls, das sich als das gewünschte 4-(Toluol-4-sulfonyloxy)-benzaldehyd-[bis-(2-phenoxy-ethyl)-acetal] (Verbindung Nr. 1 auf den beigefügten Formelseiten) erwies.

| | | | | |
|---|---|---|---|---|
| $C_{30}H_{30}O_7S$ [534,62] | ber.:<br>gef.: | C 67,40<br>C 67,7 | H 5,66<br>H 5,7 | S 6,00<br>S 5,9 |

Herstellungsbeispiel 2:

1. Stufe: 80,6 g (0,66 mol) 4-Hydroxybenzaldehyd wurden in 200 ml Tetrahydrofuran gelöst, mit 138 ml Triethylamin versetzt und auf -5 °C gekühlt. Dazu wurden 75,3 g (0,66 mol) Methansulfonsäurechlorid, gelöst in 330 ml Tetrahydrofuran und auf -5 °C vorgekühlt, bei -8 bis - 5 °C zugetropft. Es wurde 1 Stunde bei Raumtemperatur nachgerührt. Die Lösung wurde in 1000 ml destilliertes Wasser eingegossen und die Mischung mit konzentrierter Salzsäure auf pH 2 gebracht. Das ausgefallene Öl wurde in Ether aufgenommen und die wäßrige Phase mit Ether extrahiert. Die vereinigten organischen Phasen wurden mit Wasser gewaschen und getrocknet. Nach dem Einengen verblieb ein Öl, das aus Cyclohexan umkristallisiert wurde. Es wurden 84 g (63,7%) 4-Methansulfonyloxy-benzaldehyd (weißer Feststoff mit einem Schmelzpunkt von 61 bis 63 °C) erhalten.

2. Stufe: 5,2 g (0,026 mol) 4-Methansulfonyloxy-benzaldehyd wurden zusammen mit 7,6 g (0,052 mol) eines Urethanalkohols, hergestellt durch Kondensation von 1 mol Ethylencarbonat mit 1 mol n-Propylamin, 4,25 g (0,0286 mol) Orthoameisensäuretriethylester und 100 mg p-Toluolsulfonsäure im Ölbad auf 130 °C erwärmt. Dabei destillierte eine leicht bewegliche Flüssigkeit ab. Nach 2 Stunden wurde die Destillation für etwa 45 Minuten durch Anlegen eines Vakuums von 1 Torr unterstützt. Der erkaltete Rückstand wurde in Methylenchlorid aufgenommen, je zweimal mit 2%iger Natronlauge und vollentsalztem Wasser gewaschen und die organische Phase getrocknet. Nach Entfernung des Lösemittels verblieben 11,7 g (93%) 4-Methansulfonyloxy-benzaldehyd-{bis-[2-(N-propyl-carbamoyloxy)-ethyl]-acetal} (bernsteinfarbene Schmelze; Verbindung Nr. 2). Die Verbindung zeigt ein einzelnes $^1$H-NMR-Signal für die Actalgruppe bei 5,65 ppm.

Herstellungsbeispiel 3:

1. Stufe: 8,29 g (0,06 mol) 3,4-Dihydroxybenzaldehyd wurden in 40 ml Tetrahydrofuran gelöst, mit 18,5 ml Triethylamin versetzt und auf -5 °C gekühlt. Dazu wurden 22,9 g (0,12 mol) p-Toluolsulfonsäurechlorid, gelöst in 80 ml Tetrahydrofuran und auf -5 °C vorgekühlt, bei -8 bis -5 °C zugetropft. Es wurde 2 Stunden bei Raumtemperatur nachgerührt. Die Lösung wurde in 1000 ml destilliertes Wasser eingegossen und die Mischung mit konzentrierter Salzsäure auf pH 2 gebracht. Das ausgefallene Öl erstarrte nach längerem Rühren zu einem braunen Kristallbrei. Das Rohprodukt wurde abgenutscht und getrocknet. Es wurde aus Cyclohexan/Methylenchlorid unter Zusatz von Aktivkohle umkristallisiert. Es wurden 15,9 g (59,4%) 3,4-Bis-(Toluol-4-sulfonyloxy)-benzaldehyd (weißer Feststoff mit einem Schmelzpunkt von 95 bis 97 °C) erhalten.

2. Stufe: 11,6 g (0,026 mol) 3,4-Bis-(Toluol-4-sulfonyloxy)-benzaldehyd wurden zusammen mit 7,44 g (0,052 mol) N-(2-Hydroxy-ethyl)-succinimid, 4,25 g (0,0286 mol) Orthoameisensäuretriethylester und 100 mg p-Toluolsulfonsäure im Ölbad auf 130 °C erwärmt. Dabei destillierte eine leicht bewegliche Flüssigkeit ab. Nach 2 Stunden wurde die Destillation für etwa 45 Minuten durch Anlegen eines Vakuums von 1 Torr unterstützt. Der erkaltete Rückstand wurde in Methylenchlorid aufgenommen, je zweimal mit 2%iger Natronlauge und vollentsalztem Wasser gewaschen und die organische Phase getrocknet. Nach Entfernung des Lösemittels verblieben 16,9 g einer bernsteinfarbenen Schmelze, die aus Ethanol umkristallisiert wurde. Es wurden 12,8 g (90,6%) 3,4-Bis-(toluol-4-sulfonyloxy)-benzaldehyd-[bis-(2-succinimido-ethyl)-acetal] (weiße Kristalle mit einem Schmelzpunkt von 127 bis 128 °C; Verbindung Nr. 3) erhalten.

| | | | | | |
|---|---|---|---|---|---|
| $C_{33}H_{33}N_2O_{12}S_2$ [713,70] | ber.:<br>gef.: | C 55,54<br>C 55,9 | H 4,66<br>H 4,5 | N 3,92<br>N 4,0 | S 8,98<br>S 9,0 |

Herstellungsbeispiel 4:

1. Stufe: 15,4 g (0,1 mol) 3,4,5-Trihydroxybenzaldehyd wurden in 40 ml Tetrahydrofuran gelöst, mit 45,5 ml Triethylamin versetzt und auf -5 °C gekühlt. Dazu wurden 57,2 g (0,3 mol) p-Toluolsulfonsäurechlorid, gelöst in 80 ml Tetrahydrofuran und auf -5 °C vorgekühlt, bei -8 bis -5 °C zugetropft. Es wurde 2 Stunden bei Raumtemperatur nachgerührt. Die Lösung wurde in 1000 ml destilliertes Wasser eingegossen und die Mischung mit konzentrierter Salzsäure auf pH 2 gebracht. Das ausgefallene Öl erstarrte nach längerem Rühren zu einem braunen Kristallbrei. Das Rohprodukt wurde abgenutscht und getrocknet. Es wurde aus Ethylacetat unter Zusatz von Aktivkohle umkristallisiert. Es wurden 46,8 g (75,9%) 3,4,5-Tris-(toloul-4-sulfonyloxy)-benzaldehyd (weißer Feststoff mit einem Schmelzpunkt von 159 °C) erhalten.
2. Stufe: 18,5 g (0,03 mol) 3,4,5-Tris-(toluol-4-sulfonyloxy)-benzaldehyd wurden zusammen mit 5,2 g (0,035 mol) Orthoameisensäure-triethylester und 100 mg p-Toluolsulfonsäure in 150 ml Toluol bei Raumtemperatur gerührt. Es wurde eine schwache Wärmeentwicklung beobachtet. Die Mischung wurde über Nacht stehen gelassen, dann mit 8,84 g (0,065 mol) 3-Phenylpropanol sowie weiteren 100 mg p-Toluolsulfonsäure versetzt und zum Rückfluß erwärmt. Im weiteren Verlauf wurde gemäß Herstellungsbeispiel 1 verfahren. Der erkaltete Rückstand wurde in Methylenchlorid aufgenommen, je zweimal mit 2%iger Natronlauge und vollentsalztem Wasser gewaschen und die organische Phase getrocknet. Nach Entfernung des Lösemittels verblieben 21,4 g (82%) einer honigfarbenen Schmelze, die sich nicht zur Kristallisation bringen ließ. Im Dünnschichtchromatogramm (DC) zeigte sich, daß das gewünschte 3,4,5-Tris-(toluol-4-sulfonyloxy)-benzaldehyd-[bis-(3-phenyl-propyl)-acetal] (Verbindung Nr.4) eine Reinheit von mehr als 92% aufwies.

| | | | | |
|---|---|---|---|---|
| $C_{46}H_{46}O_{11}S_3$ [871,58] | ber.:<br>gef.: | C 63,39<br>C 65,0 | H 5,39<br>H 5,4 | S 11,03<br>S 10,7 |

Weitere Beispiele von erfindungsgemäßen Verbindungen sind in den beiliegenden Formelseiten aufgeführt. Ihre Synthese erfolgte analog zu literaturbekannten Verfahren. Ihre Authenzität wurde durch Elementaranalyse und $^1$H-NMR bestätigt.

Verbindung Nr. 1

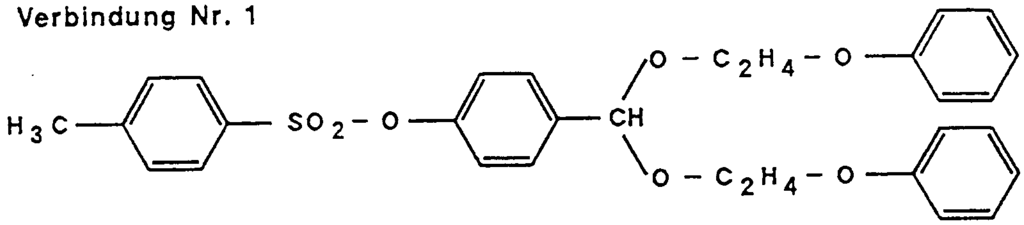

Verbindung Nr. 2

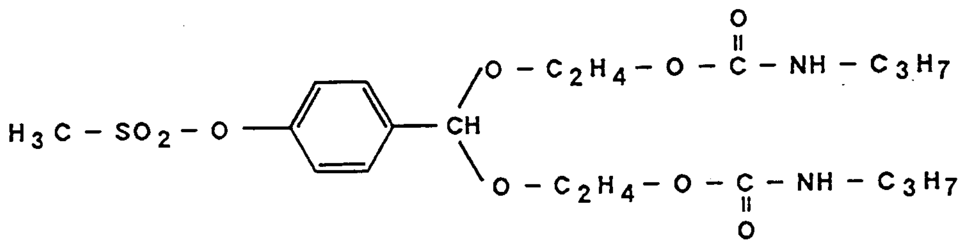

Verbindung Nr. 3

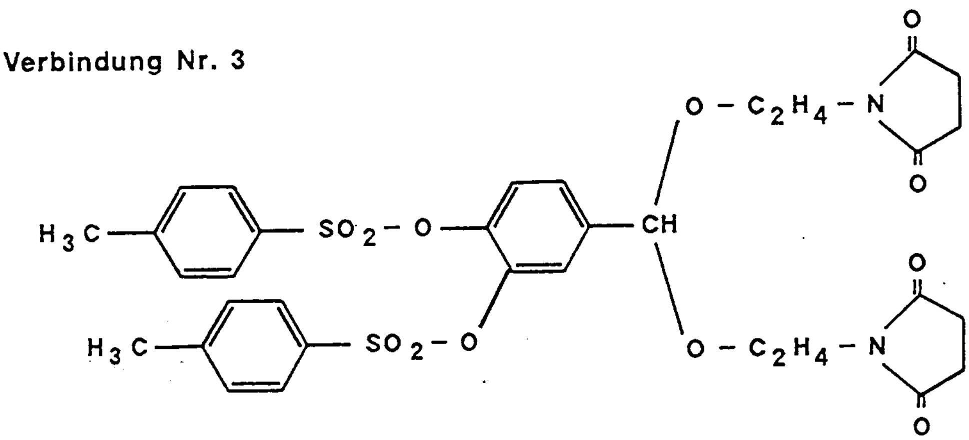

Verbindung Nr. 4

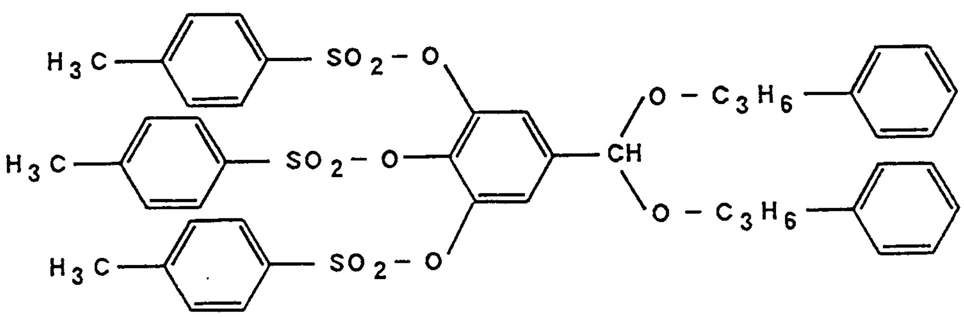

Verbindung Nr. 5

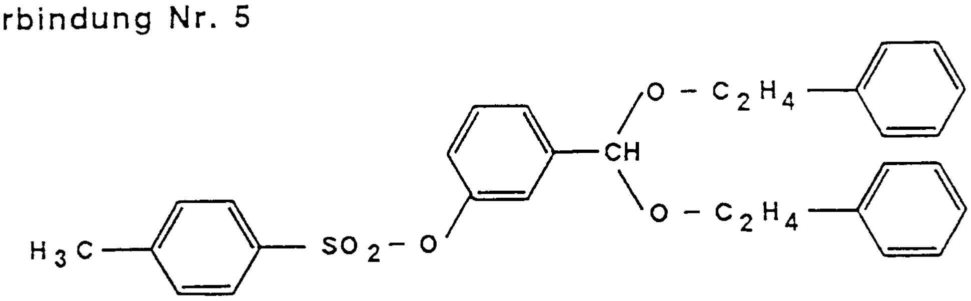

Verbindung Nr. 6

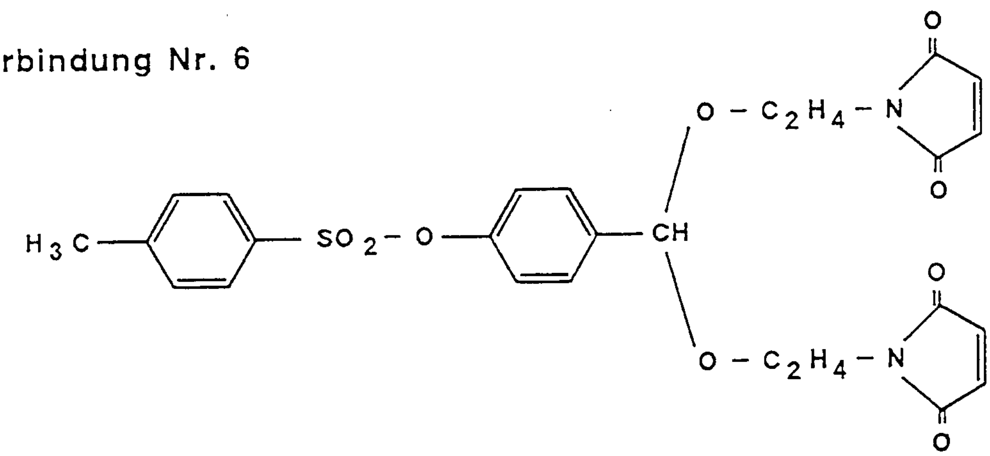

Verbindung Nr. 7

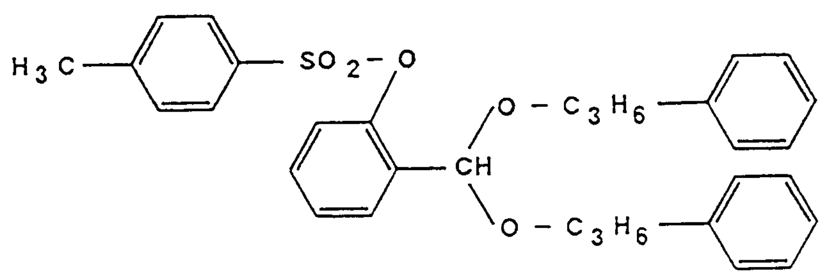

Verbindung Nr. 8

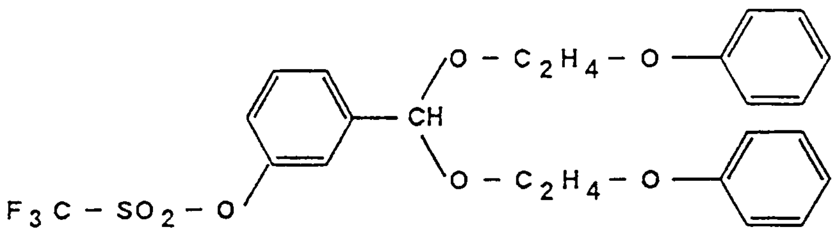

Verbindung Nr. 9

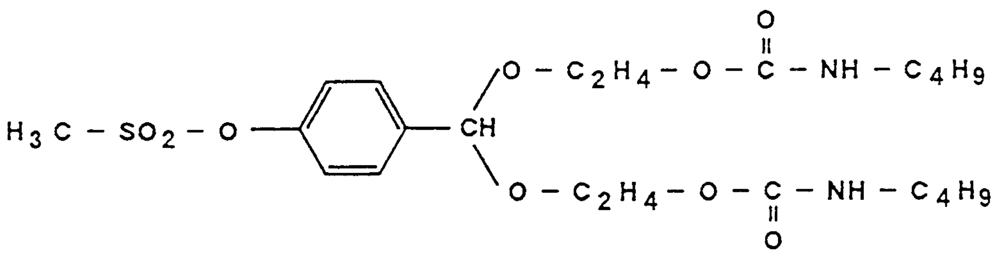

Verbindung Nr. 10

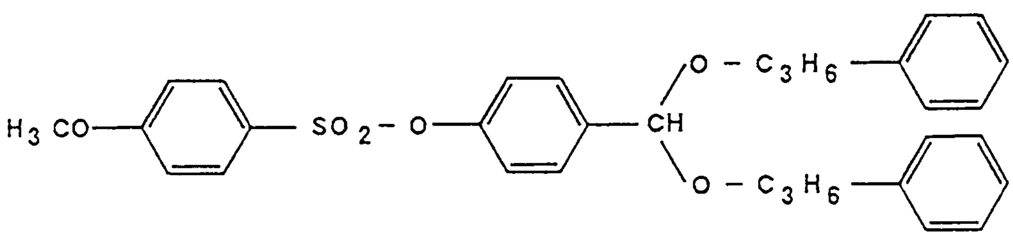

Verbindung Nr. 11

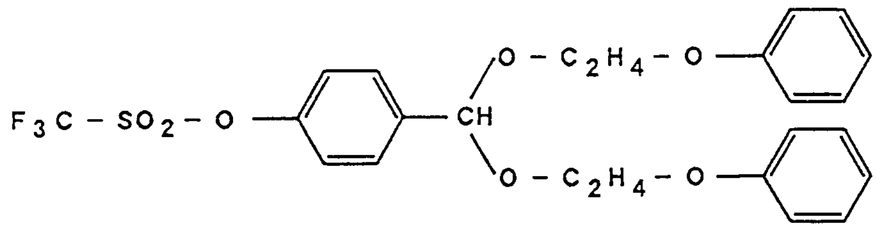

Verbindung Nr. 12

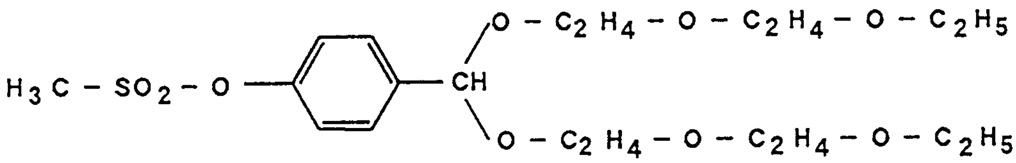

Verbindung Nr. 13

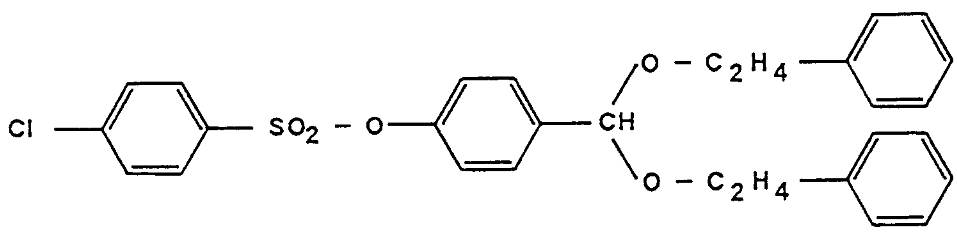

Verbindung Nr. 14

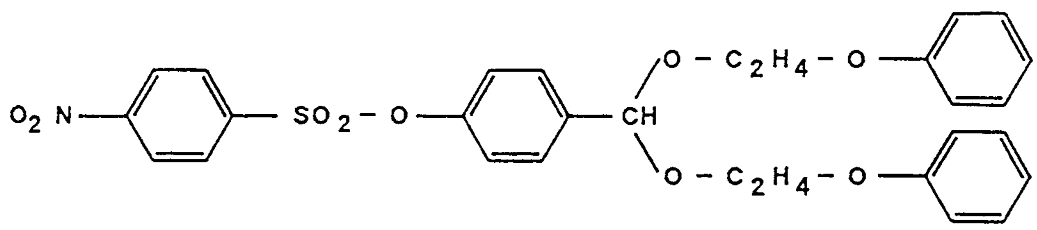

Verbindung Nr. 15

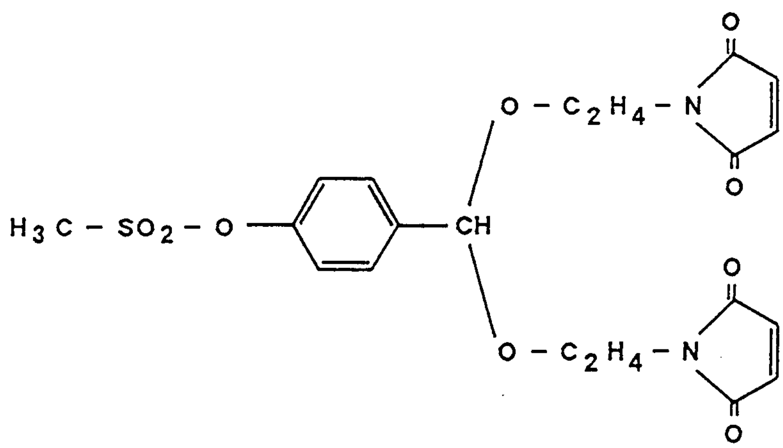

Verbindung Nr. 16

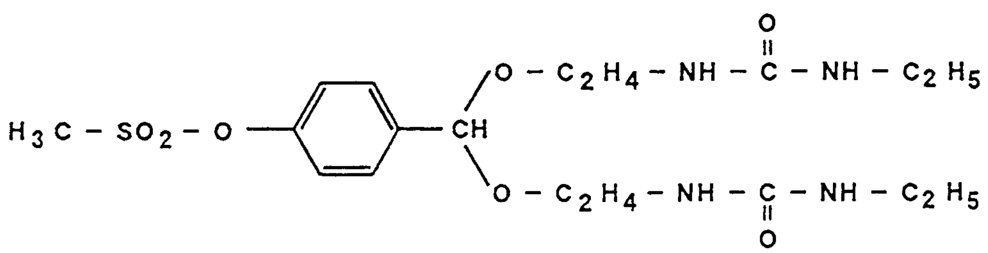

Verbindung Nr. 17

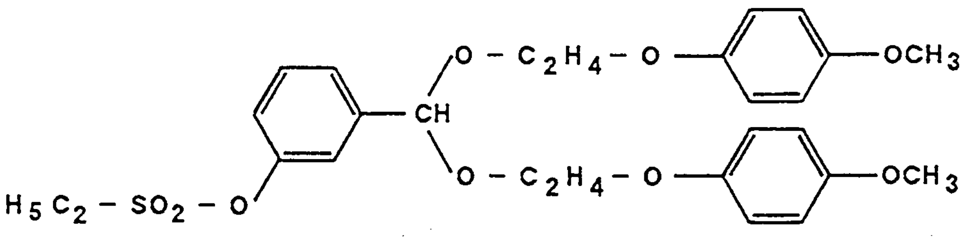

Verbindung Nr. 18

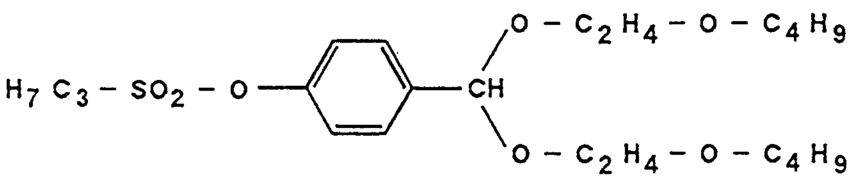

Verbindung Nr. 19

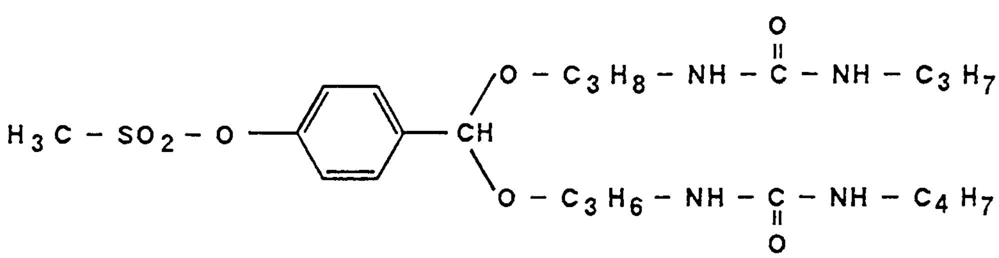

Verbindung Nr. 20

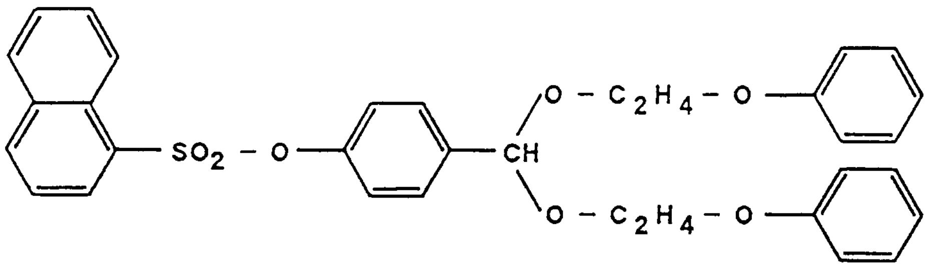

Verbindung Nr. 21

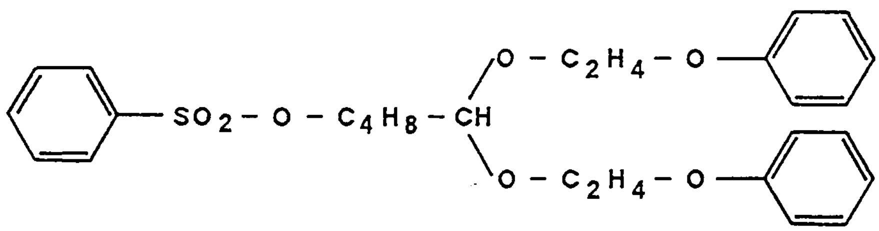

Verbindung Nr. 22

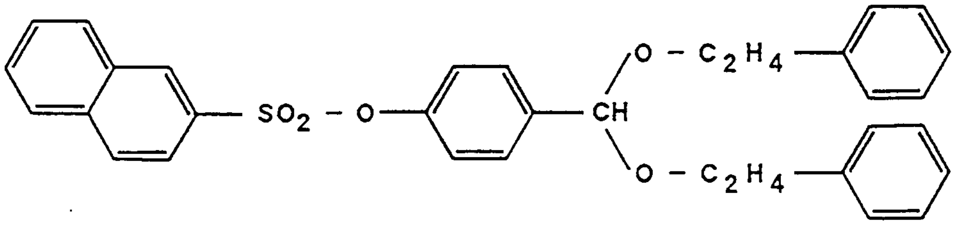

Verbindung Nr. 23

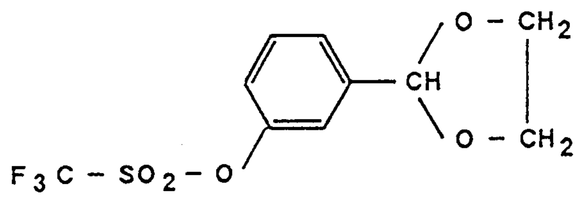

Verbindung Nr. 24

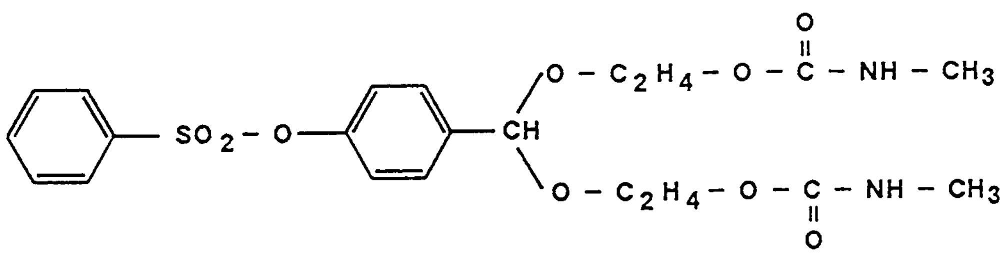

Verbindung Nr. 25

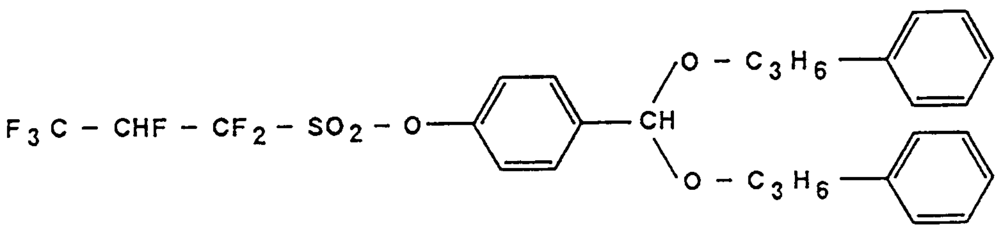

Verbindung Nr. 26

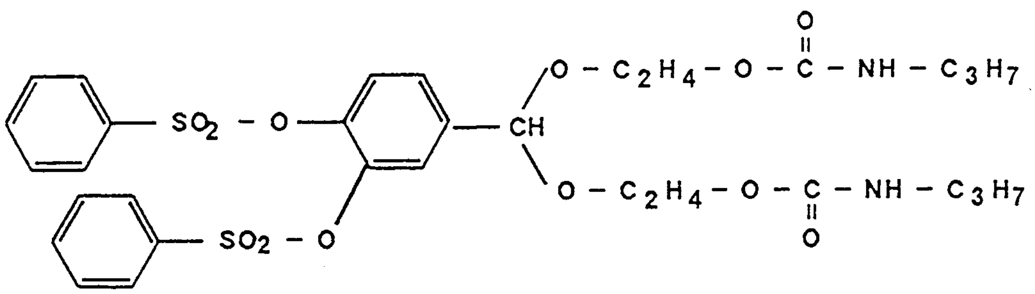

Verbindung Nr. 27

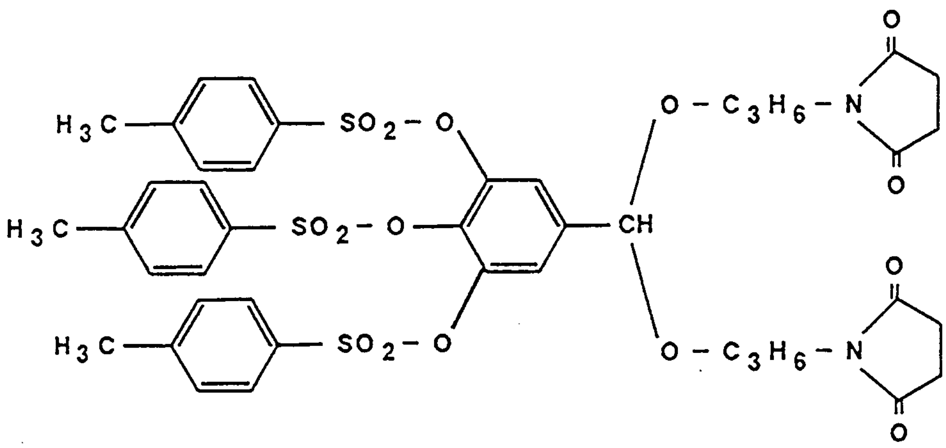

Verbindung Nr. 28

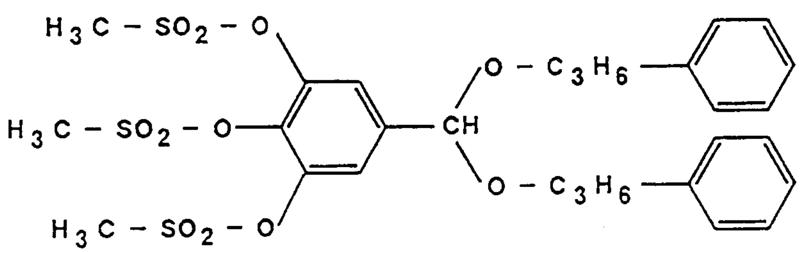

Verbindung Nr. 29

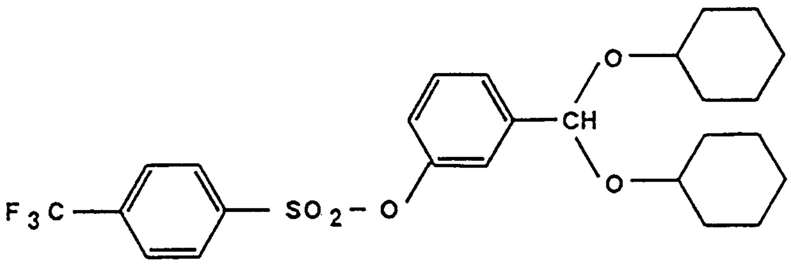

Verbindung Nr. 30

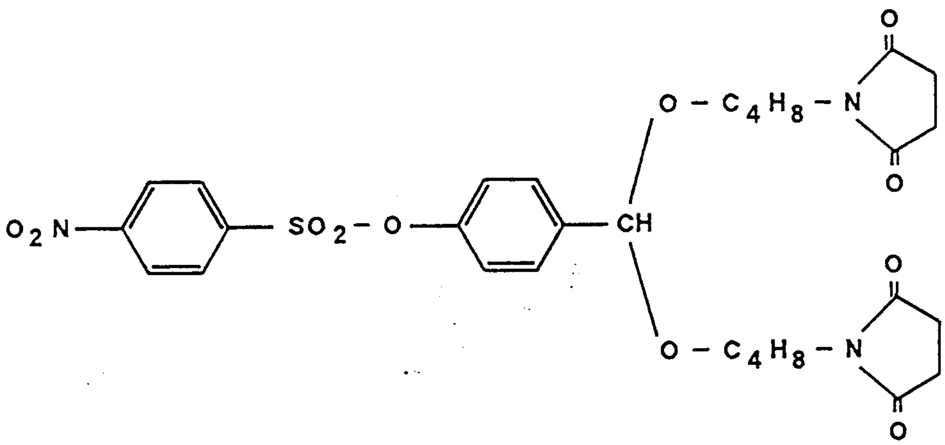

Verbindung Nr. 31

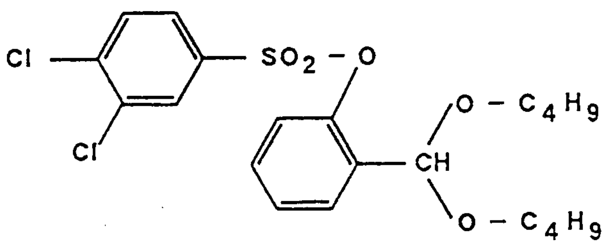

Verbindung Nr. 32

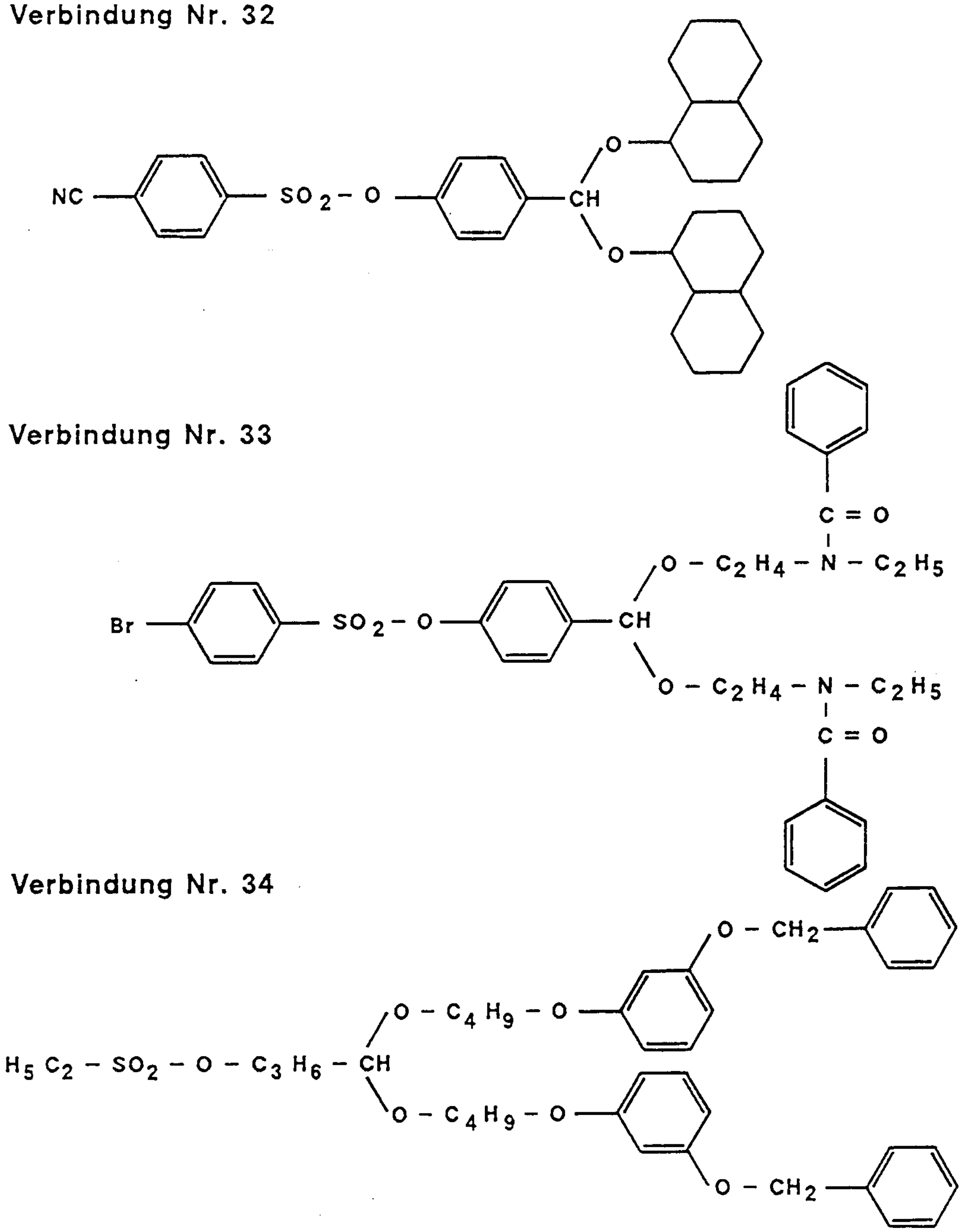

Verbindung Nr. 33

Verbindung Nr. 34

Verbindung Nr. 35

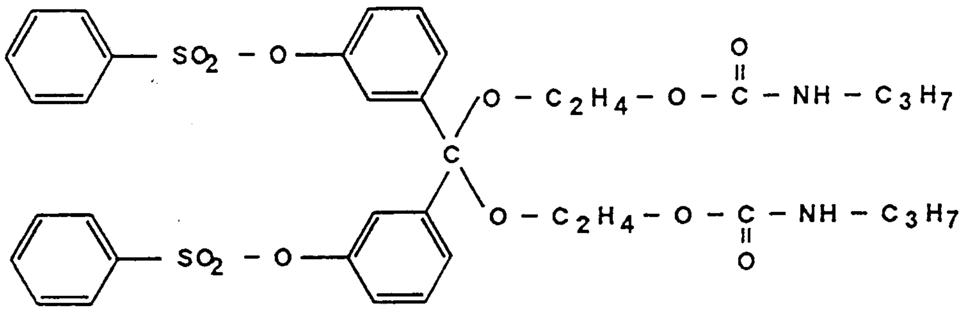

Verbindung Nr. 36

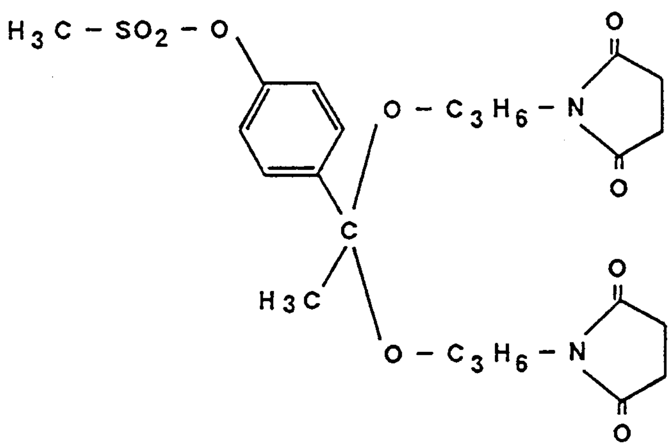

Verbindung Nr. 37

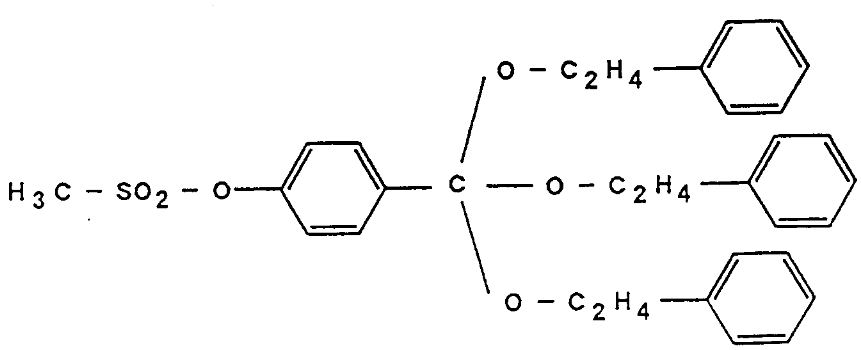

Verbindung Nr. 38

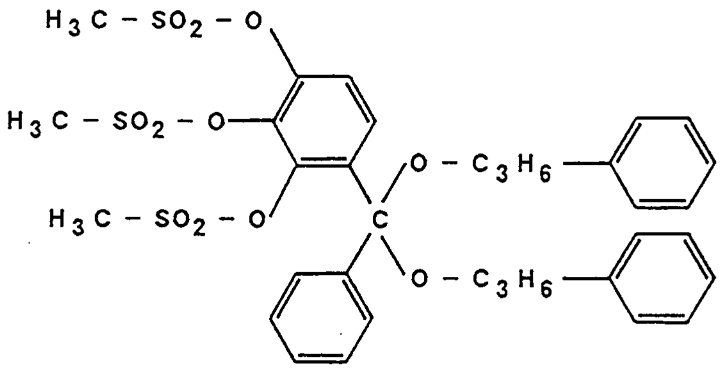

Verbindung Nr. 39

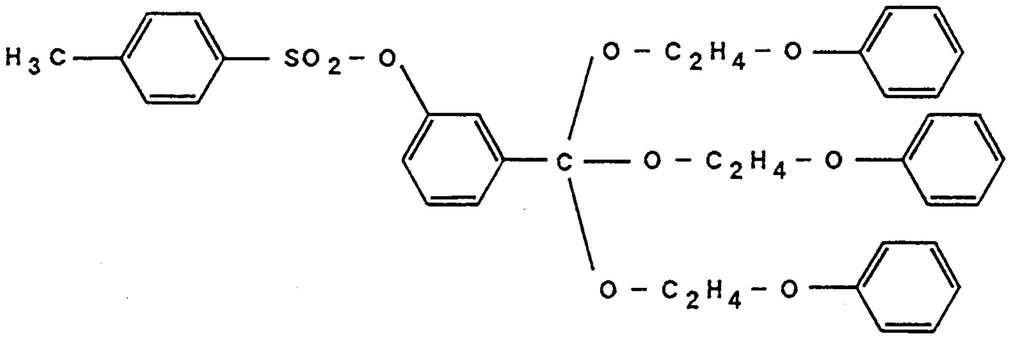

Verbindung Nr. 40

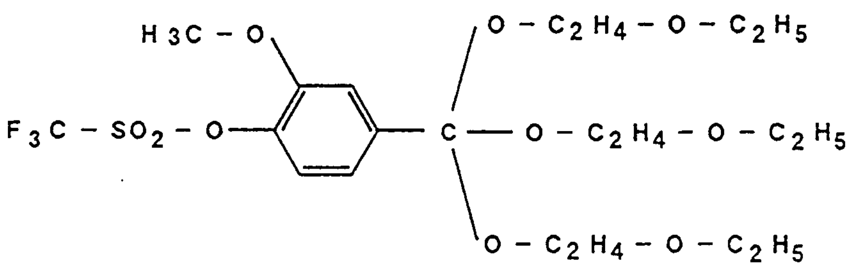

Verbindung Nr. 41

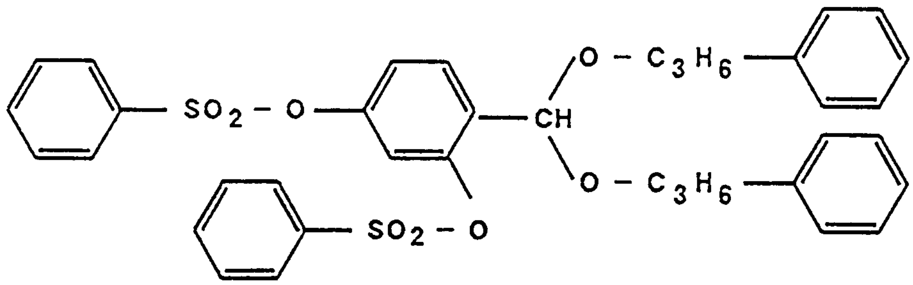

Verbindung Nr. 42

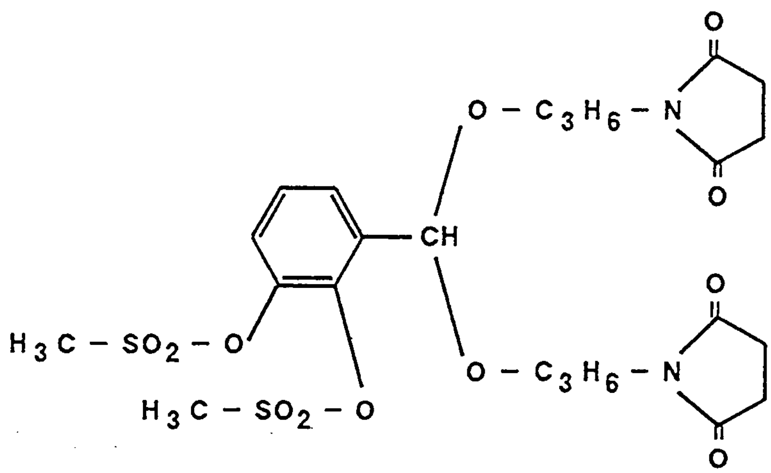

Verbindung Nr. 43

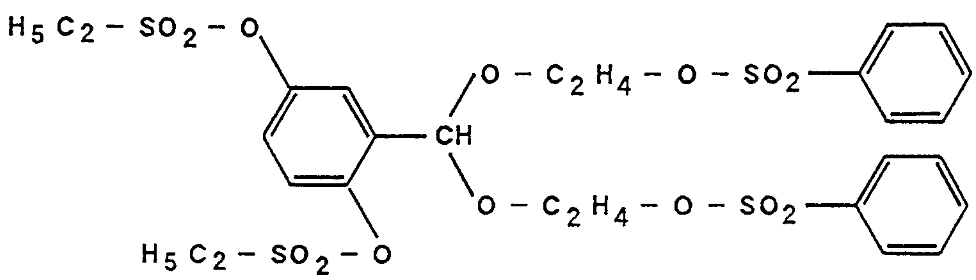

Verbindung Nr. 44

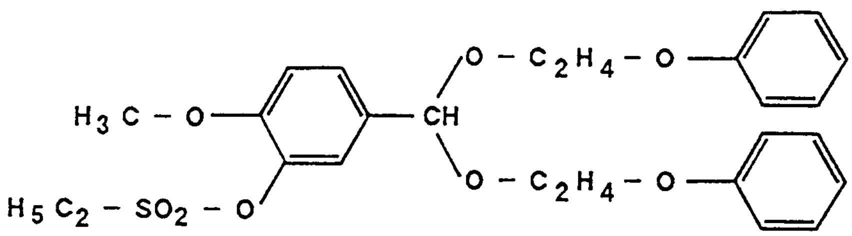

Verbindung Nr. 45

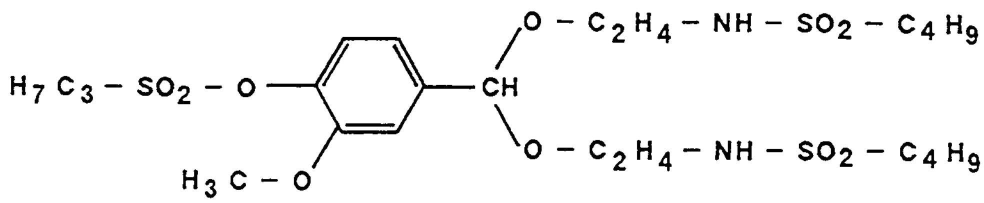

Verbindung Nr. 46

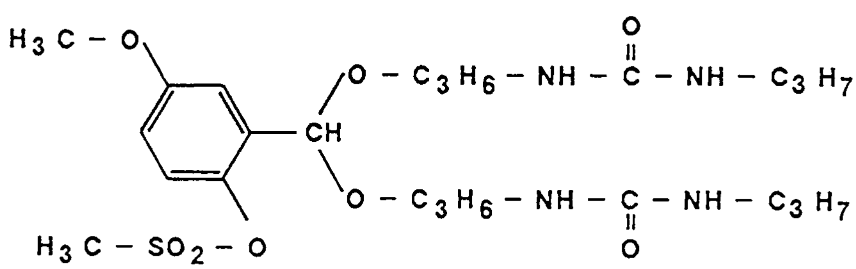

Verbindung Nr. 47

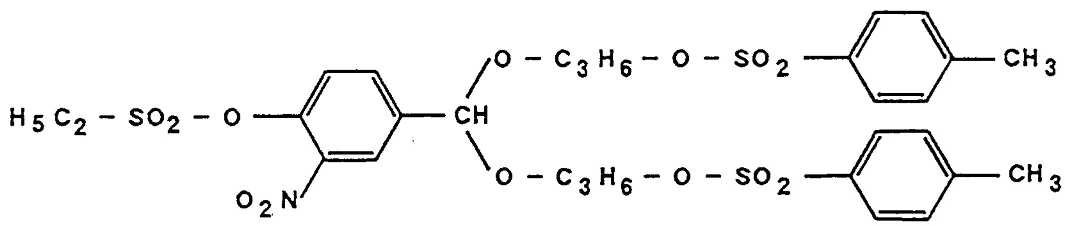

Verbindung Nr. 48

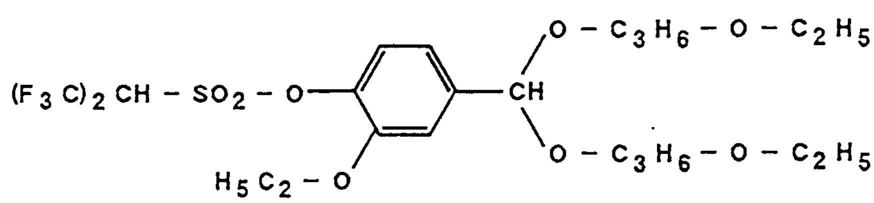

Verbindung Nr. 49

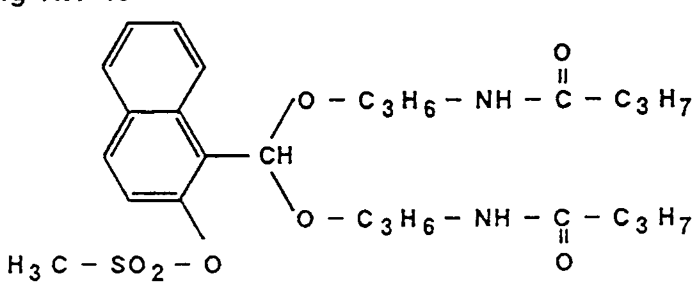

Aus der vorstehenden Auswahl erfindungsgemäßer Verbindungen der allgemeinen Formel I geht hervor, daß solche, die Acetalstrukturen aufweisen, besonders bevorzugt sind. Dies ist darauf zurückzuführen, daß ihre Zugänglichkeit sehr gut ist und sie außerdem sowohl in Lösung als auch in Schichten außerordentlich hydrolysestabil sind, und somit ein stabiles Aufzeichnungsmaterial mit diesen Verbindungen hergestellt werden kann. Den erfindungsgemäßen Acetalen liegen, wie in den Herstellungsbeispielen beschrieben, gewisse Aldehyde zugrunde, von denen als Ausgangsverbindungen folgende besonders bevorzugt sind:
2-, 3-, 4-Hydroxybenzaldehyd,
2,3-, 2,4-, 2,5-, 3,4-Dihydroxybenzaldehyd,
2,3,4-, 3,4,5-Trihydroxybenzaldehyd,
4-Hydroxy-3-methylbenzaldehyd,
2-Hydroxy-4-, 2-Hydroxy-5-, 3-Hydroxy-4-, 4-Hydroxy-3-methoxybenzaldehyd,
3,5-Dimethyl-4-, 3,4-Dimethoxy-5-, 3-Ethoxy-4-hydroxybenzaldehyd,
2-Hydroxy-5-, 3-Hydroxy-4-, 4-Hydroxy-3-, 5-Hydroxy-2-nitrobenzaldehyd,
2-Hydroxy-naphthalin-1-carbaldehyd.

Diese Aldehyde sind im Handel erhältlich, während sich andere erfindungsgemäß als Vorprodukte geeignete Hydroxyaldehyde nach den unterschiedlichsten Methoden im allgemeinen in einfacher Weise herstellen lassen. Eine Übersicht hierzu ist gegeben in Houben-Weyl, Methoden der organischen Chemie, Bd. 7/1.

Erfindungsgemäß wird ferner ein positiv arbeitendes strahlungsempfindliches Gemisch vorgeschlagen, das als wesentliche Bestandteile

a) ein in Wasser unlösliches, in wäßrig-alkalischen Lösungen lösliches oder zumindest quellbares Bindemittel und

b) eine unter Einwirkung von aktinischer Strahlung eine starke Säure bildende Verbindung mit mindestens einer durch Säure spaltbaren C-O-C-Bindung

enthält, dadurch gekennzeichnet, daß die Verbindung (b) eine Verbindung allgemeinen Formel I ist, wobei auch solche Verbindungen mit eingeschlossen sind, in denen X eine Alkylen-, Cycloalkylen- oder Arylengruppe und n = 1 bedeuten.

Die unter Verwendung der Verbindungen der allgemeinen Formel I erhaltenen erfindungsgemäßen strahlungsempfindlichen Gemische zeichnen sich durch hohe Empfindlichkeiten über einen weiten Spektralbereich aus. Ganz besonders sind sie zur Bestrahlung mit energiereicher UV-Strahlung geeignet, bevorzugt gegenüber Licht einer Wellenlänge von 190 bis 350 nm. Sie zeigen eine hohe thermische Stabilität und schaffen die Möglichkeit, auch feinste Strukturen einer Vorlage strukturgenau wiederzugeben. Durch die Belichtung werden keine korrodierenden Photolyseprodukte gebildet, so daß das Gemisch auch auf empfindlichen Substratmaterialien Verwendung finden kann.

Die im erfindungsgemäßen strahlungsempfindlichen Gemisch enthaltenen säurespaltbaren photolytischen Säurebildner können allein oder in Kombination mit anderen säurelabilen Säurebildnern der erfindungsgemäß genannten Klasse Verwendung finden. Es sind aber auch Kombinationen mit anderen photolytischen Säurebildnern möglich, wozu sich sogar die eingangs erwähnten Onium-Salze, Halogenverbindungen, insbesondere Trichlormethyltriazinderivate oder Trichlormethyloxadiazolderivate, o-Chinondiazidsulfochloride oder Organometall-Organohalogen-Kombinationen eignen. Derartige Kombinationen sind aber insgesamt nicht bevorzugt, da in solchen strahlungsempfindlichen Gemischen unter bestimmten Umständen die schon genannten Nachteile wieder auftreten können.

Der Gehalt an säurelabilen Säurebildnern der allgemeinen Formel I im erfindungsgemäßen Gemisch liegt im allgemeinen zwischen 2 und 60 Gew.-%, bevorzugt bei 5 bis 50 Gew.-%, und besonders bevorzugt bei 10 bis 40 Gew.-%, jeweils bezogen auf das Gesamtgewicht der Schicht.

Den erfindungsgemäßen Gemischen können gegebenenfalls andere säurespaltbare Verbindungen zugefügt werden; dabei haben sich vor allem folgende Verbindungsklassen bewährt:

(1) solche mit mindestens einer Orthocarbonsäureester- und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können (DE-A 26 10 842 und 29 28 636),

(2) Oligomer- oder Polymerverbindungen mit wiederkehrenden Acetal- und/oder Ketalgruppierungen in der Hauptkette (DE-A 23 06 248 und 27 18 254),

(3) Verbindungen mit mindestens einer Enolether- oder N-Acyliminocarbonatgruppierung (EP-A 0 006 626 und 0 006 627),

(4) cyclische Acetale oder Ketale von $\beta$-Ketoestern oder -amiden (EP-A 0 202 196),

(5) Verbindungen mit Silylethergruppierungen (DE-A 35 44 165 und 36 01 264),

(6) Verbindungen mit Silyl-enolethergruppierungen (DE-A 37 30 785 und 37 30 783),

(7) Monoacetale bzw. Monoketale, deren Aldehyd- bzw. Ketonkomponente eine Löslichkeit im Entwickler zwischen 0,1 und 100 g/l aufweisen (DE-A 37 30 787),

(8) Ether auf der Basis tertiärer Alkohole (US-A 4,603,101) und

(9) Carbonsäureester und Carbonate tertiärer, allylischer oder benzylischer Alkohole [US-A 4,491,628 und J. M. Fréchet et al., J. Imaging Sci. 30, 59-64 (1986)].

Es können auch Mischungen der genannten säurespaltbaren Verbindungen eingesetzt werden. Unter diesen bevorzugt verwendbar sind allerdings säurespaltbare Verbindungen, die einem der obengenannten Typen (1) bis (9) zuzuordnen sind, und darunter besonders diejenigen, die eine durch Säure spaltbare C-O-C-Bindung aufweisen. Unter diesen besonders bevorzugt sind diejenigen Verbindungen, die den Typen (1), (2), (7) und (9) angehören. Unter Typ (2) sind besonders die polymeren Acetale hervorzuheben; von den säurespaltbaren Verbindungen des Typs (7) insbesondere diejenigen, deren Aldehyd- bzw. Ketonkomponente einen Siedepunkt oberhalb 150 °C, vorzugsweise oberhalb 200 °C, aufweist. Insgesamt jedoch sind solche Mischungen nicht bevorzugt.

Die Verbindung (b) bzw. die Kombination von Verbindungen (b) ist in einer Konzentration von 2 bis 60 Gew.-% enthalten.

Das erfindungsgemäße strahlungsempfindliche Gemisch enthält ferner mindestens ein polymeres, in Wasser unlösliches, in wäßrig-alkalischen Lösungen dagegen lösliches, zumindest quellbares Bindemittel. Das Bindemittel zeichnet sich im besonderen dadurch aus, daß es mit den übrigen Bestandteilen des erfindungsgemäßen strahlungsempfindlichen Gemisches gut verträglich ist und insbesondere im Wellenlängenbereich von 190 bis 300 nm eine möglichst geringe Eigenabsorption, d.h. eine hohe Transparenz, aufweist. Bindemittel auf der Basis von Novolak-Kondensationsharzen, die in der Regel in Kombination mit Naphthochinondiaziden als photoaktive Komponenten eingesetzt werden, erfüllen diese Bedingung nicht. Zwar lassen Novolak-Kondensationsharze nach bildmäßiger Belichtung in den belichteten Bereichen eine Erhöhung der Löslichkeit gegenüber wäßrig-alkalischen Entwicklern erkennen, doch ist ihre Eigenabsorption im Bereich der für die Bestrahlung gewünschten kurzen Wellenlänge unerwünscht hoch.

Novolak-Kondensationsharze können aber in Mischung mit anderen, als Bindemittel geeigneten Harzen mit höherer Transparenz eingesetzt werden. Die Mischungsverhältnisse richten sich dabei vorwiegend nach der Art des mit dem Novolakharz zu mischenden Bindemittels. Insbesondere spielen dessen Grad an Eigenabsorption im genannten Wellenlängenbereich, aber auch die Mischbarkeit mit den anderen Bestandteilen des strahlungsempfindlichen Gemisches eine entscheidende Rolle. Im allgemeinen kann aber das Bindemittel des erfindungsgemäßen strahlungsempfindlichen Gemisches bis zu 30 Gew.-%, insbesondere bis zu 20 Gew.-%, eines Novolak-Kondensationsharzes enthalten.

Als Bindemittel geeignet sind Homo- oder Copolymere des p-Hydroxystyrols sowie seiner Alkylderivate, z. B. des 3-Methyl-4-hydroxystyrols, des 3,5-Dimethyl-4-hydroxystyrols oder des 2,3-Dimethyl-4-hydroxystyrols sowie Homo- oder Copolymere anderer Polyvinylphenole, z. B. des 2- oder 3-Hydroxystyrols, oder des 4-Methyl-3-hydroxystyrols oder die Ester der (Meth)acrylsäure mit Phenolen, z. B. Brenzkatechin, Resorcin, Hydrochinon, Pyrogallol oder Aminophenole sowie die entsprechenden Amide mit aromatischen Aminen. Als Comonomere können polymerisierbare Verbindungen wie Styrol, Methylmethacrylat, Methylacrylat oder ähnliche eingesetzt werden.

Gemische mit erhöhter Plasmabeständigkeit werden dann erhalten, wenn zur Herstellung von Copolymeren des obigen Typs Silicium enthaltende Vinylmonomere, z. B. Vinyltrimethylsilan oder Allyltrimethylsilan, verwendet werden. Die Transparenz dieser Bindemittel ist im interessierenden Bereich im allgemeinen höher, so daß eine verbesserte Strukturierung möglich ist.

Mit gleichem Erfolg lassen sich auch Homo- oder Copolymere des Maleinimids verwenden. Auch diese Bindemittel zeigen hohe Transparenz im beschriebenen Wellenlängenbereich. Als Comonomere werden auch hier bevorzugt Styrol, substituierte Styrole, Vinylphenole, Vinylether, Vinylester, Vinylsilylverbindungen oder (Meth)acrylsäureester eingesetzt.

Schließlich sind darüber hinaus auch Copolymere des Styrols mit Comonomeren verwendbar, die in wäßrig alkalischen Lösungen eine Löslichkeitserhöhung bewirken. Hierunter zählen beispielsweise Maleinsäureanhydrid, Maleinsäurehalbester oder dergleichen.

Die genannten Bindemittel können auch untereinander gemischt werden, sofern sich dadurch die optische Qualität des strahlungsempfindlichen Gemisches nicht verschlechtert. Bindemittelgemische sind jedoch nicht bevorzugt.

Die Menge des Bindemittels beträgt im allgemeinen 40 bis 98 Gew.-%, insbesondere 50 bis 95 Gew.-%, vorzugsweise 60 bis 90 Gew.-%, bezogen auf das Gesamtgewicht der strahlungsempfindlichen Mischung.

Die Extinktion des Bindemittels bzw. der Kombination von Bindemitteln (a) im Wellenlängenbereich der Empfindlichkeit der Verbindung (b) sollte weniger als 0,5 $\mu m^{-1}$ betragen.

Ferner können den erfindungsgemäßen strahlungsempfindlichen Gemischen gegebenenfalls Farbstoffe, Pigmente, Weichmacher, Netzmittel und Verlaufmittel, aber auch Polyglykole, Celluloseether, z. B. Ethylcellulose, zur Verbesserung spezieller Erfordernisse, wie Flexibilität, Haftung und Glanz, zugesetzt werden.

Soll ein Substrat beschichtet werden, so wird das erfindungsgemäße strahlungsempfindliche Gemisch zweckmäßig in einem Lösemittel oder in einer Kombination von Lösemitteln gelöst. Hierfür besonders geeignet sind Ethylenglykol, Ethylenglykolmonomethylether, Ethylenglykoldimethylether, Ethylenglykolmonoethylether oder Propylenglykolmonoalkylether, insbesondere Propylenglykolmethylether, aliphatische Ester (z. B. Ethylacetat, Hydroxyethylacetat, Alkoxyethylacetat, n-Butylacetat, Propylenglykolalkyletheracetat, insbesondere Propylenglykolmethyletheracetat oder Amylacetat), Ether (z. B. Dioxan), Ketone (z. B. Methylethylketon, Methylisobutylketon, Cyclopentanon und Cyclohexanon), Dimethylformamid, Dimethylacetamid, Hexamethylphosphorsäureamid, N-Methyl-pyrrolidon, Butyrolacton, Tetrahydrofuran und Mischungen derselben] gelöst. Besonders bevorzugt werden Glykolether, aliphatische Ester sowie Ketone. Letztendlich hängt die Wahl der Lösemittel von dem angewandten Beschichtungsverfahren, der gewünschten Schichtstärke und den Trocknungsbedingungen ab. Ebenso müssen die Lösemittel chemisch neutral sein, d. h. sie dürfen nicht mit den übrigen Schichtkomponenten irreversibel reagieren.

Die mit den genannten Lösemitteln hergestellte Lösung hat in der Regel einen Feststoffgehalt von 5 bis 60 Gew.-%, vorzugsweise bis 50 Gew.-%.

Gegenstand der Erfindung ist schließlich auch ein strahlungsempfindliches Aufzeichnungsmaterial, das im wesentlichen aus einem Substrat und einer darauf befindlichen strahlungsempfindlichen Schicht aus dem erfindungsgemäßen strahlungsempfindlichen Gemisch besteht.

Als Substrate kommen alle Materialien in Frage, aus denen Kondensatoren, Halbleiter, mehrlagige gedruckte Schaltungen oder integrierte Schaltkreise bestehen bzw. hergestellt werden können. Speziell sind Siliciumsubstrate zu nennen, die auch thermisch oxidiert und/oder mit Aluminium beschichtet, aber auch dotiert sein können. Daneben sind alle anderen in der Halbleitertechnologie üblichen Substrate möglich, wie Siliciumnitrid, Galliumarsenid und Indiumphosphid. Weiterhin kommen die aus der Flüssigkristalldisplay-Herstellung bekannten Substrate in Frage, wie z. B. Glas und Indium-Zinnoxid, ferner Metallplatten und -folien - beispielsweise aus Aluminium, Kupfer, fer, Zink-, Bimetall- und Trimetallfolien, aber auch elektrisch nichtleitende Folien, die mit Metallen bedampft sind, und Papier. Diese Substrate können thermisch vorbehandelt, oberflächlich aufgerauht, angeätzt oder, zur Verbesserung erwünschter Eigenschaften, z. B. zur Erhöhung der Hydrophilie, mit Chemikalien vorbehandelt sein.

Um der strahlungsempfindlichen Schicht einen besseren Zusammenhalt und/oder eine bessere Haftung auf der Substratoberfläche zu verleihen, kann in ihr ein Haftvermittler enthalten sein. Bei Silicium- bzw. Siliciumdioxid-Substraten kommen hierfür Haftvermittler vom Aminosilan-Typ, wie z. B. 3-Aminopropyltriethoxysilan oder Hexamethyldisilazan, in Frage.

Geeignete Träger für die Herstellung von photomechanischen Aufzeichnungsschichten, wie Druckformen für den Hochdruck, Flachdruck, Siebdruck und Flexodruck sind besonders Aluminiumplatten, die gegebenenfalls vorher anodisch oxidiert, gekörnt und/oder silikatisiert werden, daneben Zink- und Stahlplatten, die gegebenenfalls verchromt werden, sowie Kunststoffolien und Papier.

Das erfindungsgemäße Aufzeichnungsmaterial wird mit aktinischer Strahlung bildmäßig belichtet. Als Strahlungsquellen eignen sich besonders Metallhalogenidlampen, Kohlebogenlampen, Xenonlampen und Quecksilberdampflampen. Ebenso kann eine Belichtung mit energiereicher Strahlung wie Laser-, Elektronen- oder Röntgenstrahlung erfolgen. Besonders bevorzugt sind jedoch Lampen, die Licht einer Wellenlänge von 190 bis 260 nm ausstrahlen können, d.h. insbesondere Xenon- und Quecksilberdampflampen. Darüber hinaus lassen sich auch Laserlichtquellen verwenden, z. B. Excimerlaser, insbesondere KrF- oder ArF-Laser, die bei 248 bzw. 193 nm emittieren. Die Strahlungsquellen müssen in den genannten Wellenlängenbereichen eine ausreichende Emission aufweisen.

Die Stärke der lichtempfindlichen Schicht hängt vom Verwendungszweck ab. Sie beträgt im allgemeinen zwischen 0,1 und 100 $\mu$m, bevorzugt zwischen 1,0 und 10 $\mu$m.

Gegenstand der Erfindung ist ferner ein Verfahren zur Herstellung eines strahlungsempfindlichen Aufzeichnungsmaterials. Das Auftragen des strahlungsempfindlichen Gemisches auf das Substrat kann durch Aufsprühen, Fließbeschichten, Walzen, Schleuder- und Tauchbeschichten erfolgen. Danach wird das Lösemittel durch Verdampfen entfernt, so daß auf der Oberfläche des Substrats die strahlungsempfindliche Schicht zurückbleibt. Die Entfernung des Lösemittels kann durch Erhitzen der Schicht auf Temperaturen bis zu 150 °C gefördert werden. Das Gemisch kann aber auch zunächst auf obengenannte Weise auf einen Zwischenträger aufgetragen` werden, von dem aus es unter Druck und erhöhter Temperatur auf das endgültige Trägermaterial übertragen wird. Als Zwischenträger können grundsätzlich alle auch als Trägermaterialien geeigneten Materialien Anwendung finden. Anschließend wird die Schicht bildmäßig bestrahlt und zur Verbesserung der Spaltreaktion erwärmt. Danach behandelt man die Schicht mit einer Entwicklerlösung, die die bestrahlten Bereiche der Schicht löst und entfernt, so daß ein Abbild der bei der bildmäßigen Bestrahlung verwendeten Vorlage auf der Substratoberfläche verbleibt.

Als Entwickler eignen sich besonders wäßrige Lösungen, die Silikate, Metasilikate, Hydroxide, Hydrogen- und Dihydrogenphosphate, Carbonate oder Hydrogencarbonate von Alkalimetall-, Erdalkalimetall- und/oder Ammoniumionen enthalten, aber auch Ammoniak und dergleichen. Metallionenfreie Entwickler werden in der US-A 4 729 941, der EP-A 0 062 733, der US-A 4 628 023, der US-A 4 141 733, der EP-A 0 097 282 und der EP-A 0 023 758 beschrieben. Der Gehalt dieser Substanzen in der Entwicklerlösung beträgt im allgemeinen 0,1 bis 15 Gew.-%, vorzugweise 0,5 bis 5 Gew.-%, bezogen auf das Gewicht der Entwicklerlösung. Metallionenfreie Entwickler werden bevorzugt verwendet. Den Entwicklern können gegebenenfalls geringe Mengen eines Netzmittels zugesetzt sein, um die Ablösung der löslichen Bereiche der Schicht zu erleichtern.

Die entwickelten Schichtstrukturen können nachgehärtet werden. Dies geschieht im allgemeinen durch Erhitzen auf einer hot-plate bis zu einer Temperatur unterhalb der Fließtemperatur und anschließendes

ganzflächiges Belichten mit dem UV-Licht einer Xenon-Quecksilber-Dampflampe (Bereich von 200 bis 250 nm). Durch die Nachhärtung werden die Bildstrukturen vernetzt, so daß sie im allgemeinen eine Fließbeständigkeit bis zu Temperaturen von über 200 °C aufweisen. Die Nachhärtung kann auch ohne Temperaturerhöhung allein durch Bestrahlen mit energiereichem UV-Licht erfolgen.

Verwendung findet das erfindungsgemäße strahlungsempfindliche Gemisch bei der Herstellung von integrierten Schaltungen oder von diskreten elektronischen Bausteinen mit lithographischen Prozessen, da sie eine hohe Lichtempfindlichkeit aufweisen, besonders bei Bestrahlung mit Licht einer Wellenlänge zwischen 190 bis 300 nm. Da die Gemische bei Belichtung sehr gut ausbleichen, lassen sich feinere Strukturen erzielen als dies mit den bekannten Gemischen möglich ist. Die entwickelte Resistschicht dient dabei als Maske für die folgenden Prozeßschritte. Solche Schritte sind z. B. das Ätzen des Schichtträgers, das Implantieren von Ionen in den Schichtträger oder das Abscheiden von Metallen oder anderen Materialien auf dem Schichtträger.

Die folgenden Beispiele 1 bis 10 belegen die Eignung des erfindungsgemäßen Gemisches für Aufzeichnungsmaterialien in der Mikrolithographie unter Verwendung von energiereicher Strahlung. Anhand der Vergleichsbeispiele 11 und 12 wird die Überlegenheit der erfindungsgemäßen Gemische gegenüber dem Stand der Technik belegt.

In den Beispielen sind die Mengen in der Regel als Gewichtsteile (Gt) angegeben. Wenn nichts anderes angegeben ist, sind Prozentzahlen und Mengenverhältnisse in Gewichtseinheiten zu verstehen.

Beispiel 1:

Es wurde eine Beschichtungslösung hergestellt aus
6,0 Gt eines Copolymeren aus Styrol/p-Hydroxystyrol (Molverhältnis 30:70) mit einem mittleren Molekulargewicht von 27.000,
1,5 Gt eines Kresol-Formaldehyd-Novolaks mit einem Erweichungsbereich von 105 bis 120 °C und
2,0 Gt 4-(Toluol-4-sulfonyloxy)-benzaldehyd-[bis-(2-phenoxy-ethyl)-acetal] (Verbindung Nr. 1) in
42 Gt Propylenglykol-monomethylether-acetat.

Die Lösung wurde durch ein Filter mit einem Porendurchmesser von 0,2 µm filtriert und auf einen mit einem Haftvermittler (Hexamethyldisilazan) behandelten Wafer bei 3.500 Umdrehungen aufgeschleudert. Nach 1 Minute Trocknen bei 100 °C auf der hot plate wurde eine Schichtdicke von 1,0 µm erhalten.

Das Aufzeichnungsmaterial wurde bildmäßig unter einer Vorlage mit der UV-Strahlung einer Xenon-Quecksilberdampflampe (Filter mit Transmission von 240 bis 260 nm) mit einer Energie von 80 $mJ/cm^2$ belichtet und vor der Entwicklung etwa 30 Minuten bei Raumtemperatur gelagert.

Entwickelt wurde das Aufzeichnungsmaterial mit einem 0,3 n alkalischen Entwickler folgender Zusammensetzung:

5,3 Gt Natriummetasilikat x 9 $H_2O$,
3,4 Gt Trinatriumphosphat x 12 $H_2O$,
0,3 Gt Natriumdihydrogenphosphat und
191 Gt vollentsalztes Wasser.

Nach einer Entwicklungsdauer von 60 s erhielt man ein fehlerfreies Abbild der Maske mit steilen Resistflanken, wobei auch Strukturen < 0,7 µm detailgetreu aufgelöst waren. Eine Untersuchung der Flanken der Resistprofile mittels Rasterelektronenmikroskopie belegte, daß diese praktisch senkrecht zur Substratoberfläche ausgerichtet waren.

Beispiel 2:

Es wurde eine Beschichtungslösung hergestellt aus
7,5 Gt eines Copolymeren aus Styrol/p-Hydroxystyrol (Molverhältnis 20:80) mit einem mittleren Molekulargewicht von 32.000 und
2,0 Gt 3,4-Bis-(toluol-4-sulfonyloxy)-benzaldehyd-[bis-(2-succinimido-ethyl)-acetal] (Verbindung Nr. 3) in 42 Gt Propylenglykol-monomethylether-acetat.

Die Lösung wurde durch ein Filter mit einem Porendurchmesser von 0,2 µm filtriert und auf einen mit einem Haftvermittler (Hexamethyldisilazan) behandelten Wafer bei 3.000 Umdrehungen aufgeschleudert. Nach 1 Minute Trocknen bei 100 °C auf der hot plate wurde eine Schichtdicke von 1,22 µm erhalten.

Das Aufzeichnungsmaterial wurde bildmäßig unter einer Vorlage mit der UV-Strahlung einer Xenon-Quecksilberdampflampe bei 240 bis 260 nm mit einer Energie von 76 $mJ/cm^2$ belichtet, etwa 30 Minuten bei Raumtemperatur gelagert und anschließend mit dem in Beispiel 1 beschriebenen Entwickler verarbeitet.

Nach einer Entwicklungsdauer von 60 s erhielt man ein fehlerfreies Abbild der Maske mit hoher Flankenstabilität, wobei auch hier Strukturen < 0,7 µm detailgetreu aufgelöst waren.

Beispiel 3:

Ein entsprechend Beispiel 1 hergestellter Wafer wurde unter einer Vorlage mit KrF-Excimer-Laser-Strahlung einer Wellenlänge von 248 nm mit einer Energie von 100 $mJ/cm^2$ bestrahlt. Nach der Entwicklung wurde ein originalgetreues Abbild der Vorlage erhalten.

Beispiel 4:

Es wurde eine Beschichtungslösung hergestellt aus
7,5 Gt des in Beispiel 2 angegebenen Copolymeren und
2,0 Gt 4-(Toluol-4-sulfonyloxy)-benzaldehyd-[bis-(2-phenoxy-ethyl)-acetal] (Verbindung Nr. 1) in 42 Gt Propylenglykol-monomethylether-acetat.

Die Lösung wurde durch ein Filter mit einem Porendurchmesser von 0,2 µm filtriert und auf insgesamt zwei mit einem Haftvermittler (Hexamethyldisilazan) behandelte Wafer bei 3.100 Umdrehungen aufgeschleudert. Nach 1 Minute Trocknen bei 100 °C auf der hot plate wurde in beiden Fällen eine Schichtdicke von 1,08 µm erhalten.

Einer der beschichteten Wafer wurde bildmäßig unter einer Vorlage mit der UV-Strahlung einer Xenon-Quecksilberdampflampe bei 240 bis 260 nm mit einer Energie von 80 $mJ/cm^2$ belichtet, 75 s bei 100 °C erwärmt, und anschließend mit einem Entwickler verarbeitet, der aus 3 % Tetramethylammoniumhydroxid und 97 % entionisiertem Wasser bestand.

Nach einer Entwicklungsdauer von 60 s erhielt man ein fehlerfreies Abbild der Maske mit hoher Flankenstabilität, wobei auch hier Strukturen < 0,7 µm detailgetreu aufgelöst waren.

Der zweite Wafer wurde nach 14 Tagen wie oben beschrieben belichtet, getempert und entwickelt. Es wurden praktisch die gleichen Ergebnisse erzielt wie vorstehend beschrieben. Dies bedeutet, daß das Gemisch in getrockneter Form auf einem Substrat aufgetragen eine ausgezeichnete Stabilität aufweist.

Beispiel 5:

Es wurde eine Beschichtungslösung hergestellt aus
7,5 Gt eines Homopolymeren aus 3-Methyl-4-hydroxystyrol mit einem mittleren Molekulargewicht von 25.000 und
2,0 Gt 4-(Toluol-4-sulfonyloxy)-benzaldehyd-[bis-(2-phenoxy-ethyl)-acetal] (Verbindung Nr. 1) in
42 Gt Propylenglykol-monomethylether-acetat.

Die Lösung wurde durch ein Filter mit einem Porendurchmesser von 0,2 µm filtriert, und in zwei gleiche Teile getrennt. Der eine Teil wurde auf einen mit einem Haftvermittler (Hexamethyldisilazan) behandelten Wafer bei 3.100 Umdrehungen aufgeschleudert. Nach 1 Minute Trocknen bei 100 °C wurde eine Schichtdicke von 1,08 µm erhalten.

Das Aufzeichnungsmaterial wurde bildmäßig unter einer Vorlage mit der Strahlung einer Xenon-Quecksilberdampflampe bei 240 bis 260 nm mit einer Energie von 60 $mJ/cm^2$ belichtet, 75 s bei 100 °C erwärmt und anschließend mit dem in Beispiel 4 beschriebenen Entwickler verarbeitet.

Nach einer Entwicklungsdauer von 60 s erhielt man ein fehlerfreies Abbild der Maske mit hoher Flankenstabilität, wobei auch hier Strukturen < 0,7 µm detailgetreu aufgelöst waren.

Der zweite Teil wurde nach 20 Wochen Lagerung im Kühlschrank der gleichen Prozedur unterzogen. Es wurden identische Ergebnisse erhalten, woraus hervorgeht, daß das Gemisch in Lösung außerordentlich gute Stabilität aufweist.

Beispiel 6:

Es wurde eine Beschichtungslösung hergestellt aus
7,5 Gt des in Beispiel 2 angegebenen Copolymeren und
2,0 Gt 4-Methansulfonyloxy-benzaldehyd-{bis-[2-(N-propyl-carbamoyloxy)-ethyl]-acetal} (Verbindung Nr. 2) in
42 Gt Propylenglykol-monomethylether-acetat.

Die Lösung wurde durch ein Filter mit einem Porendurchmesser von 0,2 µm filtriert und auf einen mit einem Haftvermittler (Hexamethyldisilazan) behandelten Wafer bei 3.100 Umdrehungen aufgeschleudert.

Nach 1 Minute Trocknen bei 100 °C wurde eine Schichtdicke von 1,08 µm erhalten.

Das Aufzeichnungsmaterial wurde bildmäßig unter einer Vorlage mit der UV-Strahlung einer Xenon-Quecksilberdampflampe bei 240 bis 260 nm mit einer Energie von 60 mJ/cm$^2$ belichtet, für 75 Sekunden bei 100 °C erwärmt, und anschließend mit dem in Beispiel 1 beschriebenen Entwickler verarbeitet.

Nach einer Entwicklungsdauer von 60 s erhielt man ein fehlerfreies Abbild der Maske mit hoher Flankenstabilität, wobei auch hier Strukturen < 0,7 µm detailgetreu aufgelöst waren.

Beispiel 7:

Der Versuch von Beispiel 4 wurde zweimal wiederholt, wobei jedoch nicht 60 s lang bei 100 °C erwärmt wurde, sondern 90 s lang bei 90 °C bzw. 60 s lang bei 110 °C. In beiden Fällen waren die Ergebnisse praktisch gleich und stimmten mit den im Beispiel 4 beschriebenen überein , was bedeutet, daß das erfindungsgemäße Aufzeichnungsmaterial einen großen Verarbeitungsspielraum aufweist.

Beispiel 8:

Es wurde eine Beschichtungslösung hergestellt aus
7,5 Gt eines Copolymeren aus Styrol und Maleimid (Molverhältnis 1:1) mit einem Erweichungsbereich von 165 bis 180 °C und
2,0 Gt 3,4,5-Tris-(toluol-4-sulfonyloxy)-benzaldehyd-[bis-(3-phenyl-propyl)-acetal] (Verbindung Nr.4) in
42 Gt Cyclohexanon.

Die Lösung wurde durch ein Filter mit einem Porendurchmesser von 0,2 µm filtriert und auf einen mit einem Haftvermittler (Hexamethyldisilazan) behandelten Wafer bei 3.700 Umdrehungen aufgeschleudert. Nach 1 Minute Trocknen bei 100 °C wurde eine Schichtdicke von 0,98 µm erhalten.

Das Aufzeichnungsmaterial wurde bildmäßig unter einer Vorlage mit der Strahlung einer Xenon-Quecksilberdampflampe bei 240 bis 260 nm mit einer Energie von 120 mJ/cm$^2$ belichtet und 60 s auf 90 °C erwärmt.

Entwickelt wurde das Aufzeichnungsmaterial mit einer 0,02 n wäßrigen Lösung von Tetramethylammoniumhydroxid, wobei die belichteten Bereiche innerhalb von 60 s rückstandsfrei abgelöst wurden.

Ein weiteres Mal wurde ein fehlerfreies Abbild der Maske mit steilen Resistflanken erhalten. Der Dunkelabtrag betrug weiniger als 20 nm; auch Strukturen kleiner als 0,7 µm waren detailgetreu aufgelöst.

Beispiel 9:

Es wurde eine Beschichtungslösung hergestellt aus
7,5 Gt des in Beispiel 8 angegebenen Copolymeren und
2,0 Gt 2-(Toluol-4-sulfonyloxy)-benzaldehyd-[bis-(3-phenyl-propyl)-acetal] (Verbindung Nr. 7) in
42 Gt Propylenglykol-monomethylether-acetat.

Die Lösung wurde durch ein Filter mit einem Porendurchmesser von 0,2 µm filtriert und auf einen mit einem Haftvermittler (Hexamethyldisilazan) behandelten Wafer bei 3.500 Umdrehungen aufgeschleudert. Nach 1 Minute Trocknen bei 100 °C wurde eine Schichtdicke von 1,00 µm erhalten.

Das Aufzeichnungsmaterial wurde bildmäßig unter einer Vorlage mit der Strahlung einer Xenon-Quecksilberdampflampe bei 240 bis 260 nm mit einer Energie von 67 mJ/cm$^2$ belichtet und 60 s auf 110 °C erwärmt.

Entwickelt wurde das Aufzeichnungsmaterial mit einer 0,02 n wäßrigen Lösung von Tetramethylammoniumhydroxid, wobei die belichteten Bereiche innerhalb von 60 s rückstandsfrei abgelöst wurden und ein detailgetreues Abbild der Vorlage erhalten wurde. Die Kantensteilheit des Bildes war ausgezeichnet.

Beispiel 10:

Es wurde eine Beschichtungslösung hergestellt aus
7,5 Gt des in Beispiel 2 beschriebenen Copolymeren und 2,0 Gt 4-Methansulfonyloxy-orthobenzoesäure-(tris-phenethyl-ester) (Verbindung Nr. 37) in
42 Gt Propylenglykol-monomethylether-acetat.

Die Lösung wurde durch ein Filter mit einem Porendurchmesser von 0,2 µm filtriert und auf einen mit einem Haftvermittler (Hexamethyldisilazan) behandelten Wafer bei 3.500 Umdrehungen aufgeschleudert. Nach 1 Minute Trocknen bei 100 °C wurde eine Schichtdicke von 1,04 µm erhalten.

Das Aufzeichnungsmaterial wurde bildmäßig unter einer Vorlage mit der Strahlung einer Xenon-Quecksilberdampflampe bei 260 nm mit einer Energie von 45 $mJ/cm^2$ belichtet und anschließend 20 Minuten bei Raumtemperatur gelagert.

Entwickelt wurde das Aufzeichnungsmaterial mit einer 0,27 n wäßrigen Lösung von Tetramethylammoniumhydroxid, wobei die belichteten Bereiche innerhalb von 60 s rückstandsfrei abgelöst wurden und ein detailgetreues Abbild der Vorlage erhalten wurde. Linien und Spalten bis herab zu 0,5 µm wurden maskengetreu wiedergegeben. Es zeigte sich, daß die gefertigte Lösung des Materials auch nach 6-wöchiger Lagerung im Dunkeln noch reproduzierbare lithographische Ergebnisse lieferte, die mit den ersten Versuchen identisch waren.

Beispiele 11 und 12 (Vergleichsbeispiele):

Die Resistformulierung des Beispiels 2 wurde derart abgeändert, daß anstelle von 2,0 Gt der Verbindung Nr. 3 0,2 Gt der ebenfalls als Säurebildner bekannten Verbindungen Triphenylsulfonium-hexafluorphosphat (Beispiel 11) bzw. 2-Nitrobenzyltosylat (Beispiel 12) zugesetzt wurden.

Nach der Beschichtung und Trocknung zeigte sich, daß die Formulierung des Beispiels 11 gegenüber KrF-Excimer-Laserlicht einer Wellenlänge von 248 nm unter identischen Prozeßbedingungen um etwa 10% empfindlicher ist, als die erfindungsgemäße Mischung des Beispiels 2, während sich die Empfindlichkeit der Formulierung des Beispiels 12 nicht von der des Beispiels 2 unterschied.

Bei Verwendung des Onium-Salzes (Beispiel 11) wurden allerdings Strukturen mit sogenanntem "Lackfuß" erhalten, d.h. Resistreste haften in den belichteten Bereichen am Substrat an, während bei Verwendung des Tosylesters (Beispiel 12) Oberflächenvernetzungen sichtbar waren, die die freigelegten Substratflächen teilweise überdachten. In beiden Fällen waren somit keine akzeptablen Strukturierungen erhältlich.

## Patentansprüche

**1.** Verbindungen der allgemeinen Formel I

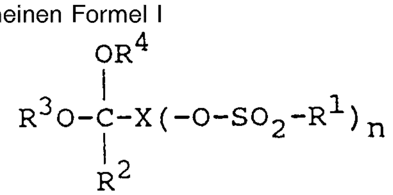

(I)

worin

| | |
|---|---|
| $R^1$ | einen Alkyl-, hochfluorierten Alkyl-, Perfluoralkyl- oder Arylrest, |
| $R^2$ | ein Wasserstoffatom, einen Alkyl- oder Arylrest, eine der Gruppen $(R^1-SO_2-O-)_nX-$ oder $R^3O-$, |
| $R^3$ und $R^4$ | gleich oder verschieden sind und Alkyl-, Cycloalkylalkyl-, Cycloalkenylalkyl- oder Aralkylreste, in denen 1 bis 3 aliphatische $CH_2$- oder CH-Gruppen durch $NR^5$, O, S, CO, CO-O, CO-NH, O-CO-NH, CO-NH-CO, NH-CO-NH, $SO_2$, $SO_2$-O, $SO_2$-NH ersetzt sein können; Alkenyl-, Alkinyl-, Cycloalkyl- oder Cycloalkenylreste bedeuten oder untereinander zu einem heterocyclischen Ring verbunden sind, |
| $R^5$ | einen Acylrest, |
| X | eine Alkantriyl-, Cycloalkantriyl- oder Arentriylgruppe oder eine Alkantetrayl-, Cycloalkantetrayl- oder Arentetraylgruppe und |
| n | 2 oder 3 |

bedeuten.

**2.** Verbindungen nach Anspruch 1, dadurch gekennzeichnet, daß $R^1$ einen Alkyl-, Perfluoralkyl- oder hochfluorierten Alkylrest mit 1 bis 6 Kohlenstoffatomen, einen Arylrest mit 6 bis 12 Kohlenstoffatomen und $R^2$ ein Wasserstoffatom oder einen Rest $R^3O$ bedeutet.

**3.** Verbindungen nach Anspruch 1 oder 2, dadurch gekennzeichnet, daß $R^3$ und $R^4$ gleich oder verschieden sind und Alkyl- oder Arylalkylgruppen mit bis zu 15 Kohlenstoffatomen bedeuten.

**4.** Positiv arbeitendes strahlungsempfindliches Gemisch, das im wesentlichen

a) ein in Wasser unlösliches, in wäßrig-alkalischen Lösungen lösliches oder zumindest quellbares Bindemittel und

b) eine unter Einwirkung von aktinischer Strahlung eine starke Säure bildende Verbindung mit mindestens einer durch Säure spaltbaren C-O-C-Bindung

enthält, dadurch gekennzeichnet, daß die Verbindung (b) eine Verbindung gemäß einem der Ansprüche 1 bis 3 ist, wobei auch solche Verbindungen mit eingeschlossen sind, in denen X eine Alkylen-, Cycloalkylen- oder Arylengruppe und n = 1 bedeuten.

**5.** Strahlungsempfindliches Gemisch nach Anspruch 4, dadurch gekennzeichnet, daß X eine Alkylengruppe mit 2 bis 10 Kohlenstoffatomen, eine Cycloalkylengruppe mit 4 bis 10 Kohlenstoffatomen oder eine Arylengruppe mit 6 bis 12 Kohlenstoffatomen bedeutet.

**6.** Strahlungsempfindliches Gemisch nach Anspruch 4, dadurch gekennzeichnet, daß die Verbindung (b) gegenüber Licht einer Wellenlänge von 190 bis 350 nm empfindlich ist.

**7.** Strahlungsempfindliches Gemisch nach Anspruch 4, dadurch gekennzeichnet, daß es die Verbindung (b) in einer Konzentration von 2 bis 60 Gew.-% enthält.

**8.** Strahlungsempfindliches Gemisch nach Anspruch 4, dadurch gekennzeichnet, daß das Bindemittel (a) im Wellenlängenbereich der Empfindlichkeit der Verbindung (b) eine Extinktion von weniger als 0,5 $\mu m^{-1}$ aufweist.

**9.** Strahlungsempfindliches Gemisch nach Anspruch 8, dadurch gekennzeichnet, daß das Bindemittel ein Polymeres mit phenolischen Hydroxylgruppen ist.

**10.** Strahlungsempfindliches Gemisch nach Anspruch 4, dadurch gekennzeichnet, daß es das Bindemittel (a) in einer Konzentration von 40 bis 98 Gew.-% enthält.

**11.** Strahlungsempfindliches Aufzeichnungsmaterial aus einem Schichtträger und einer strahlungsempfindlichen Schicht, dadurch gekennzeichnet, daß die Schicht aus einem strahlungsempfindlichen Gemisch gemäß einem der Ansprüche 4 bis 10 besteht.

## Claims

**1.** A compound of the formula I

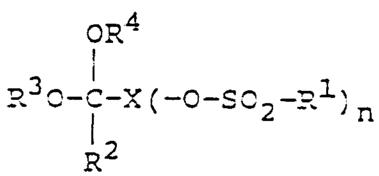

(I)

in which

| | |
|---|---|
| $R^1$ | is an alkyl, highly fluorinated alkyl, perfluoroalkyl or aryl radical, |
| $R^2$ | is a hydrogen atom, an alkyl or aryl radical or one of the groups $(R^1\text{-}SO_2\text{-}O\text{-})_nX\text{-}$ or $R^3O\text{-}$, |
| $R^3$ and $R^4$ | are identical or different and are alkyl, cycloalkylalkyl, cycloalkenylalkyl or aralkyl radicals, in which 1 to 3 aliphatic $CH_2$ or CH groups can be replaced by $NR^5$, O, S, CO, CO-O, CO-NH, O-CO-NH, CO-NH-CO, NH-CO-NH, $SO_2$, $SO_2$-O or $SO_2$-NH, or alkenyl, alkynyl, cycloalkyl or cycloalkenyl radicals or are mutually linked to form a heterocyclic ring, |
| $R^5$ | is an acyl radical, |
| X | is an alkanetriyl, cycloalkanetriyl or arenetriyl group or an alkanetetrayl, cycloalkanetetrayl or arenetetrayl group and |
| n | is an integer from 1 to 3 |

**2.** A compound as claimed in claim 1, wherein $R^1$ is an alkyl, perfluoroalkyl or highly fluorinated alkyl radical having 1 to 6 carbon atoms, an aryl radical having 6 to 12 carbon atoms and $R^2$ is a hydrogen atom or an $R^3O$ radical.

**3.** A compound as claimed in claim 1 or 2, wherein $R^3$ and $R^4$ are identical or different and are alkyl or arylalkyl groups having up to 15 carbon atoms.

**4.** A positive-working radiation-sensitive mixture which contains essentially
a) a binder which is insoluble in water and soluble or at least swellable in aqueous alkaline solutions, and
b) a compound which generates a strong acid under the action of actinic radiation and which has at least one acid-cleavable C-O-C bond,
wherein the compound (b) is a compound as claimed in any of claims 1 to 3, comprising also compounds in which X is an alkylene, cycloalkylene or arylene group and in which n is 1.

**5.** A radiation-sensitive mixture as claimed in claim 4, wherein X is an alkylene group having 2 to 10 carbon atoms, a cycloalkylene group having 4 to 10 carbon atoms or an arylene group having 6 to 12 carbon atoms.

**6.** A radiation-sensitive mixture as claimed in claim 4, wherein the compound (b) is sensitive to light of a wavelength from 190 to 350 nm.

**7.** A radiation-sensitive mixture as claimed in claim 4, which contains the compound (b) in a concentration from 2 to 60 % by weight.

**8.** A radiation-sensitive mixture as claimed in claim 4, wherein the binder (a) has an extinction of less than 0.5 $\mu m^{-1}$ in the wavelength region of the sensitivity of compound (b).

**9.** A radiation-sensitive mixture as claimed in claim 8, wherein the binder is a polymer having phenolic hydroxy groups.

**10.** A radiation-sensitive mixture as claimed in claim 4, which contains the binder (a) in a concentration from 40 to 98 % by weight.

**11.** A radiation-sensitive recording material comprising a layer support and a radiation-sensitive layer, wherein the layer is composed of a radiation-sensitive mixture as claimed in any of claims 4 to 10.

**Revendications**

**1.** Composés de formule générale I

$$R^3O-\overset{\displaystyle OR^4}{\underset{\displaystyle R^2}{\overset{|}{\underset{|}{C}}}}-X(-O-SO_2-R^1)_n \qquad (I)$$

dans laquelle

$R^1$ représente un radical alkyle, alkyle hautement fluoré, perfluoroalkyle ou aryle,

$R^2$ représente un atome d'hydrogène ou un radical alkyle ou aryle, l'un des groupes $(R^1-SO_2-O-)_nX-$ ou $R^3O-$,

$R^3$ et $R^4$ sont identiques ou différents et représentent des radicaux alkyle, cycloalkylalkyle, cycloalcénylalkyle ou aralkyle, dans lesquels 1 à 3 groupes aliphatiques $CH_2$ ou CH peuvent être remplacés par $NR^5$, O, S, CO, CO-O, CO-NH, O-CO-NH, CO-NH-CO, NH-CO-NH, $SO_2$, $SO_2$-O, $SO_2$-NH; des radicaux alcényle, alcynyle, cycloalkyle ou cycloalcényle, ou sont liés l'un à l'autre pour former un cycle hétérocyclique,

$R^5$ représente un radical acyle,

X représente un groupe alcanetriyle, cycloalcanetriyle ou arènetriyle, ou bien un groupe

alcanetétra-yle, cycloalcanetétra-yle ou arènetétra-yle et
n est 2 ou 3.

**2.** Composés selon la revendication 1, caractérisés en ce que $R^1$ représente un radical alkyle, perfluoroalkyle ou alkyle hautement fluoré ayant de 1 à 6 atomes de carbone, un radical aryle ayant de 6 à 12 atomes de carbone, et $R^2$ représente un atome d'hydrogène ou un radical $R^3O$.

**3.** Composés selon la revendication 1 ou 2, caractérisés en ce que $R^3$ et $R^4$ sont identiques ou différents et représentent des groupes alkyle ou arylalkyle ayant jusqu'à 15 atomes de carbone.

**4.** Composition sensible aux radiations, travaillant en positif, qui contient essentiellement
a) un liant insoluble dans l'eau, soluble ou au moins susceptible de gonfler dans des solutions aqueusesalcalines, et
b) un composé comportant au moins une liaison C-O-C pouvant être rompue par un acide, et formant un acide fort sous l'effet d'un rayonnement actinique,
caractérisée en ce que le composé (b) est un composé selon l'une des revendications 1 à 3, les composés dans lesquels X représente un groupe alkylène, cycloalkylène ou arylène et n = 1 étant également inclus.

**5.** Composition sensible aux radiations selon la revendication 4, caractérisée en ce que X représente un groupe alkylène ayant de 2 à 10 atomes de carbone, un groupe cycloalkylène ayant de 4 à 10 atomes de carbone ou un groupe arylène ayant de 6 à 12 atomes de carbone.

**6.** Composition sensible aux radiations selon la revendication 4, caractérisée en ce que le composé (b) est sensible à une lumière de longueur d'onde de 190 à 350 nm.

**7.** Composition sensible aux radiations selon la revendication 4, caractérisée en ce qu'elle contient le composé (b) à une concentration de 2 à 60 % en poids.

**8.** Composition sensible aux radiations selon la revendication 4, caractérisée en ce que le liant (a) présente une extinction de moins de 0,5 $\mu m^{-1}$ dans la région de longueur d'onde de la sensibilité du composé (b).

**9.** Composition sensible aux radiations selon la revendication 8, caractérisée en ce que le liant est un polymère à groupes hydroxy phénoliques.

**10.** Composition sensible aux radiations selon la revendication 4, caractérisée en ce qu'elle contient le liant (a) à une concentration de 40 à 98 % en poids.

**11.** Matériau de reprographie sensible aux radiations, composé d'un support de couche et d'une couche sensible aux radiations, caractérisé en ce que la couche consiste en une composition sensible aux radiations selon l'une des revendications 4 à 10.